# EUROPEAN PATENT SPECIFICATION

(11) **EP 2 474 533 B1**
(45) Date of publication and mention of the grant of the patent: **25.02.2015**
(21) Application number: 10780057.5
(22) Date of filing: 24.05.2010
(51) Int. Cl.: C07D 213/64, A61K 31/4412, A61P 43/00

(54) **PREPARATION OF 1-(SUBSTITUTED BENZYL)-5-TRIFLUOROMETHYL-2-(1H)PYRIDONE COMPOUNDS AND SALTS THEREOF AND THEIR APPLICATIONS**
HERSTELLUNG VON 1-(SUBSTITUIERTE BENZYL)-5-TRIFLUORMETHYL-2-(1H)PYRIDON-VERBINDUNGEN UND IHRER SALZE UND DEREN VERWENDUNG
PRÉPARATION DE COMPOSÉS DE 1-(BENZYL SUBSTITUÉ)-5-TRIFLUOROMÉTHYL-2-(1H)PYRIDONE ET DE LEURS SELS, ET LEUR UTILISATION

(30) Priority: 25.05.2009 CN 200910043502
(43) Date of publication of application: 11.07.2012
(73) Proprietor: Central South University, Hunan 410013 (CN)
(72) Inventor: HU, Gaoyun, Changsha Hunan 410013 (CN); TAO, Lijian, Changsha Hunan 410013 (CN); CHEN, Jun, Changsha Hunan 410013 (CN)
(74) Representative: Bassil, Nicholas Charles
(86) International application number: PCT/CN2010/073131
(87) International publication number: WO 2010/135976

(56) References cited:
- WO-A1-2008/154207
- WO-A2-2005/009392
- CN-A- 1 646 125
- CN-A- 1 878 757
- CN-A- 101 235 030
- CN-A- 101 237 869
- DATABASE REGISTRY [Online] CHEMICAL ABSTRACTS SERVICE, COLUMBUS, OHIO, US; 30 May 2001 (2001-05-30), XP002684268, Database accession no. 339024-94-1
- DATABASE REGISTRY [Online] CHEMICAL ABSTRACTS SERVICE, COLUMBUS, OHIO, US; 18 October 2004 (2004-10-18), XP002684269, Database accession no. 764691-46-5
- DATABASE REGISTRY [Online] CHEMICAL ABSTRACTS SERVICE, COLUMBUS, OHIO, US; 23 November 2004 (2004-11-23), XP002684270, Database accession no. 786724-02-5
- DATABASE REGISTRY [Online] CHEMICAL ABSTRACTS SERVICE, COLUMBUS, OHIO, US; 25 May 2004 (2004-05-25), XP002684271, Database accession no. 685542-68-1

## Description

### TECHNICAL FIELD

The invention relates to 1-(substituted benzyl)-5-trifluoromethyl-2(1H)pyridone compounds, preparation methods and medical applications for the same.

### BACKGROUND OF THE INVENTION

Fibrosis is in a variety of organs or tissues to cause reduction of parenchyma cells therein and increase of fibrous connective tissues, while eventually bringing damage of tissue structures, dysfunction or even organ failure. Mechanism, diagnostic methods and prevention measures for fibrosis of organs or tissues have been widely studied. In prior art, a considerable progress is made in some aspects, but some key issues unresolved still exist.
US patents US3839346A, US4052509A, US4042699 disclose 29 pyridone compounds having structural formula I as follows, 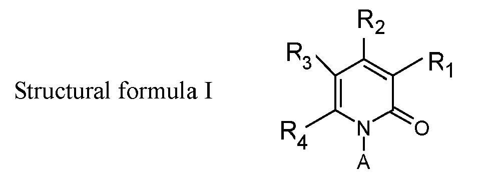 and disclose functions of the pyridone compounds of resisting inflammation, allaying fever, reducing level of serum uric acid, relieving pain or the like, wherein 5-methyl-1-phenyl-2(1H)-pyridone (Pirfenidone, PFD) has a best activity and a lower toxicity.
US patent US5,310,562 discloses 5-methyl-1-phenyl-2(1H)-pyridone for the first time in 1994, that is Pirfenidone (PFD), having an anti-fibrosis biological activity; subsequently US patent US5,518,729 and US5,716,632 disclose N-substituted-2-(1H)pyridone described as the structural formula I and N-substituted-3-(1H)pyridone having the same anti-fibrosis function. 44 compounds are specified, most of which are known compounds derived from US patent US4052509; and in the compounds, R1, R2, R3, and R4 are defined as methyl group or ethyl group.
Pirfenidone (PFD) is proven to have effectiveness in fibrosis prevention through in vitro and animal experiments. The pirfenidone has functions of stopping or even converting ECM accumulation and preventing or reversing fibrosis and scar formation in the experiments of animals with renal fibrosis and pulmonary fibrosis and in the clinical treatment of patients with idiopathic pulmonary fibrosis (Shimizu T, Fukagawa M, Kuroda T, et al. Pirfenidone prevents collagen accumulation in the remnant kidney in rats with partial nephrectomy. Kidney Int, 1997,52 (Suppl 63): 5239-243 ; Raghu G, Johnson WC, Lockhart D, et al. Treatment of idiopathic pulmonary fibrosis with a new antifibrotic agent, pirfenidone. Am J Respir Crit Care Med, 1999,159 : 1061-1069) .
The applicant proposes a CN patent ZL02114190.8 and provides a class of pyridone compounds shown in the structural formula II. 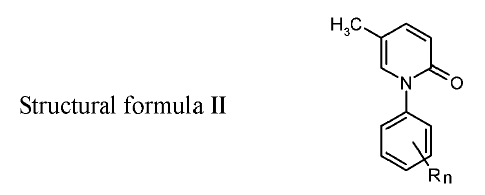 if n=1, substituent R is F, Br, or I;
if n=2, substituent R is F, Cl, Br, I, saturated linear alkyl group, oxo-substituted saturated linear alkyl group, or halo-substituted saturated linear alkyl group.
The substituent R is either at ortho-position, meta-position, para-position or the like on a benzene ring.
Pirfenidone has come into the market in Japan in 2008 for treating indications for pulmonary fibrosis, however Pirfenidone and its derivatives have strength not high enough. The clinical dose of Pirfenidone achieves 2400mg/day.
Patents WO2007053685 and WO2006122154 disclose compounds having functions of inhibiting p38 kinase, applied to treatment of fibrosis diseases and figured in the structural formula III; 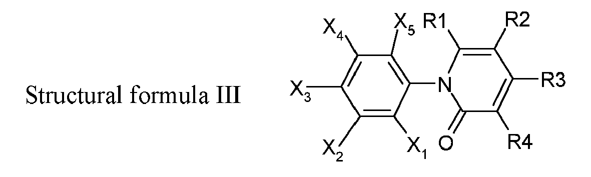 alkyl , substituted alkyl , alkenyl , haloalkyl, nitroalkyl , hydroxyalkyl, alkoxyl , phenyl , substituted phenyl , halogen, hydroxyl , alkoxyalkyl , carboxyl , alkoxycarbonyl , etc.; X1-X5 each are H, halogen, alkoxyl group, or hydroxyl group.
WO2007062167 also discloses compounds having functions of inhibiting p38 kinase and applied to treatment of various fibrosis diseases, wherein some structures are shown as follows: 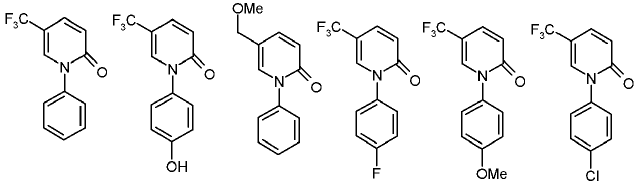 Some simple substituents are provided on the benzene rings of the compounds.
CN patent 200710034357 discloses some similar compounds having the above structures with anti-fibrosis activity and a compound with the anti-fibrosis activity shown in the structural formula IV

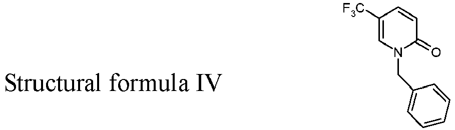

Those compounds are provided with TFM at 5-position of the pyridone ring with no any substitutents on aromatic ring of phenyl group, thereby overcoming the disadvantages of inferior action of Pirfenidone.
DE patent DE4343528 reports a class of compounds having insecticidal actions in agriculture, with the structural formula V as follows. 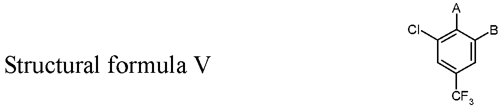 in structural formula V, A and B are substituted by various heterocyclic rings, such as furan ring, imidazole, pyridine and pyridone; wherein a class of compounds with the structural formula VI is included.

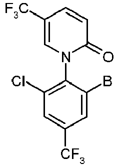

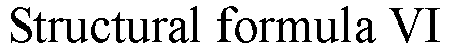

EP patents EP259048, EP367410 and EP398499 report a class of compounds having insecticidal actions in agriculture, with the structural formula VII as follows: 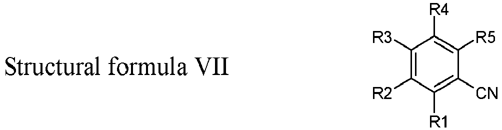 wherein a class of compounds having the structural formula VIII, in which R₁ is pyridone and R₁₀ is O or S, is included.

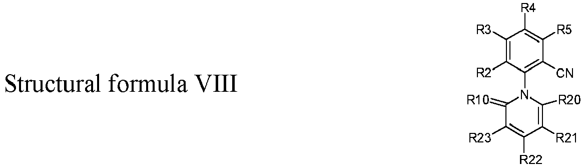

EP patent EP216541 reports a class of compounds having insecticidal actions in agriculture, with the structural formula IX as follows: 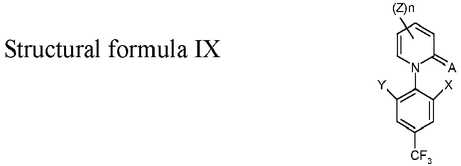 wherein a class of compounds with the structural formula X is included.

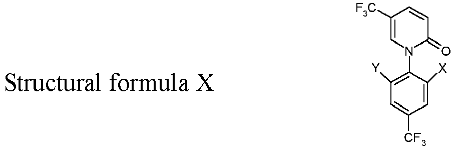

EP patent EP488220 reports a class of compounds having insecticidal actions, with the structural formula XI as follows:

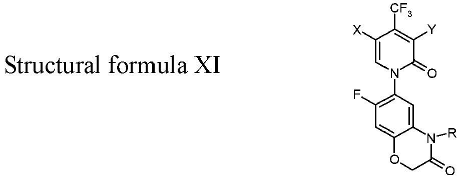

In structures of the above-mentioned compounds, the pyridine ring and the benzene ring at 1-position of the pyridine ring have a plurality of substituents; the compounds with complicated structures have not been reported to have the anti-fibrosis function. In the meanwhile, more fluorine atoms in the structure will result in stronger lipid solubility of the molecule.
DE102004027359 discloses a class of compounds capable of adjusting dopamine-3 receptor and applied to treatment of Parkinson's disease and schizophrenosis; 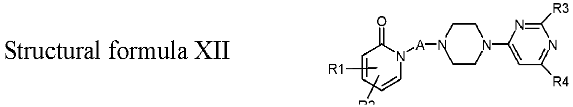 wherein, A is a hydrocarbon chain with 4-6 atoms, having 1-2 substituted methyl groups thereon; or 1-2 carbon atoms in the carbon chain are substituted by O, C=O, S and other atoms; R1 and R2 are H, CN, NO2, halogen atom, OR⁵, NR⁶R⁷, C(O)*_{N}*R⁶R⁷, O-C(O)*_{N}*R⁶R⁷; C1-C6 alkyl, C1-C6 haloalkyl, etc.

### SUMMARY OF THE INVENTION

The invention provides 1-(substituted benzyl)-5-trifluoromethyl-2-(1H)pyridine compounds shown in structural formula XIII, 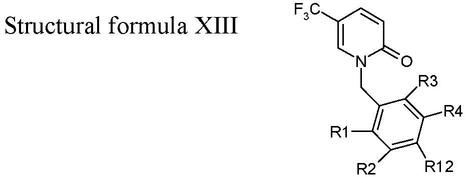 wherein R1-R4, R12 are selected from H, NO₂, hydroxyl, amino, halogen atom, C₁-C₆ alkoxyl, NR¹⁰R¹¹³, OR¹³, C(O)R¹⁴, O-C(O)R¹⁴ , C₁-C₆ alkyl, C₁-C₆ haloalkyl, and C₂-C₆ alkenyl ; wherein R1∼R4, R12 are not simultaneously H, R¹⁴ is selected from C₁-C₆ alkyl; where in NR¹⁰R¹¹, OR¹³, R¹⁰ and R¹¹ are selected from H, C₁-C₆ hydroxyalkyl, esterified C₁-C₆ hydroxyalkyl, C₁-C₆ alkoxyalkyl, or structural formula XIV, and R¹⁰ and R¹¹ are not simultaneously H; R¹³ is selected from hydroxyalkyl, alkoxyalkyl. 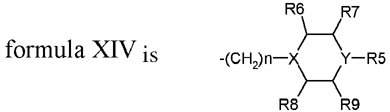 wherein R5 is selected from H, C₁-C₆ alkyl, C₁-C₆ haloalkyl, C₁-C₆ hydroxyalkyl, and C₂-C₆ alkenyl; R6-R9 are selected from H, C₁-C₆ alkoxyl, =O, C₁-C₄ alkyl, C₁-C₄ haloalkyl, C₁-C₄ hydroxyalkyl, and C₂-C₄ alkenyl; X is selected from N and CH ; Y is selected from N, O,and CH; and when Y is O, then R5 is absent; and n is 1- 6 and pharmaceutically available salts, including hydrochlorate, sulfate, phosphate, perchlorate, methanesulfonate, trifluoromethanesulfonate, formate, acetate, propionate, butyrate, maleate, succinate, trifluoroacetate, succinate, salicylate, DL-aspartate, D-aspartate, L-aspartate, DL-glutamate, D-glutamate, L-glutamate, glycerate, succinate, stearate, DL-tartrate, D-tartrate, L-tartrate, (+/-)-mandelate, (R)-(-)-mandelate, (S)-(+)-mandelate, citrate, mucate, maleate, malonate, benzoate, DL-malate, D-malate, L-malate, hemimalate, 1-adamantane acetate, 1-adamantane carboxylate, flavianate, sulfoacetate, (+/-)-lactate, L-(+)-lactate, D-(-)-lactate, pamoate, D-α-galacturonic acid salt, glycerate, DL-cystine salt, D-cystine salt, L-cystine salt, DL-homocystine salt, D-homocystine salt, L-homocystine salt, DL-cysteine salt, D-cysteine salt, L-cysteine salt, (4S)-hydroxy-L-proline, cyclopropane-1,1-dicarboxylate, 2,2-methyl malonate, tyrosine salt, proline salt, fumarate, 1-hydroxy-2-naphthoate, phosphonoacetate, carbonate, bicarbonate, 3-phosphonopropionate, DL-pyroglutamate, D-pyroglutamate, L-pyroglutamate, toluenesulfonate, benzenesulfonate, esilate, (+/-)-camsilate, naphthalenesulfenesulfonate, 1R-(-)-camsilate, 1S-(+)-camsilate, 1,5-napadisilate, 1,2-ethanedisulphonate, 1,3-propanedisulphonate, 3-(N-morpholino) propane sulphonate, biphenyl sulphonate, isethionate, 1-hydroxy-2-naphthalenesulfenesulfonate, dihydric phosphate, potassium hydrogen phosphate, dipotassium phosphate, potassium phosphate, sodium hydrogen phosphate, disodium phosphate, sodium phosphate, sodium dihydrogen phosphate, calcium phosphate, tertiary calcium phosphate, hexafluoro phosphate, ethenyl phosphate, 2-carboxylethyl phosphate and phenyl phosphate.
More preferably, one of R1-R4 and R12 is NR¹⁰R¹¹ or OR¹³.

According to embodiments of the invention, more preferably, others are H if one of R1∼R4 and R12 is NR¹⁰R¹¹ or OR¹³_{.}

According to embodiments of the invention, the following compounds are preferred:
1-(4-nitrobenzyl)-5-(trifluoromethyl)pyridin-2(1H)-one;
1-(4-amino-benzyl)-5-(trifluoromethyl)pyridin-2(1H)-one;
1-(4-((3-morpholinylpropyl)amino)benzyl)-5-(trifluoromethyl)pyridin-2(1H)-one;
1-(4-(((3-piperidin-1-yl)propyl)amino)benzyl)-5-(trifluoromethyl)pyridin-2(1H)-one;
1-(4-((3-(4-methyl-piperazin-1-yl)propyl)amino)benzyl)-5-(trifluoromethyl)pyridin-2(1H)-on e;
1-(4-((2-hydroxyethyl)amino)benzyl)-5-(trifluoromethyl)pyridin-2(1H)-one;
1-(4-((2-(piperidyl-1-yl)ethyl)amino)benzyl)-5-(trifluoromethyl)pyridin-2(1H)-one;
1-(4-((2-morpholinylethyl)amino)benzyl)-5-(trifluoromethyl)pyridin-2(1H)-one;
1-(4-((2-(4-methyl-piperazin-1-yl)ethyl)amino)benzyl)-5-(trifluoromethyl)pyridin-2(1H)-one;
1-(4-((2-(piperazin-1-yl)ethyl)amino)benzyl)-5-(trifluoromethyl)pyridin-2(1H)-one;
1-(4-((2-(4-(2-hydroxyethyl)piperazin-1-yl)ethyl)amino)benzyl)-5-(trifluoromethyl)pyridin-2 (1H)-one;
1-(4-((2-(4-methyl-piperazin-1-yl)ethyl)amino)benzyl)-5-(trifluoromethyl)pyridin-2(1H)-one dihydrochloride;
1-(4-acetamide-benzyl)-5-(trifluoromethyl)pyridin-2(1H)-one;
1-(2,6-dichlorobenzyl)-5-(trifluoromethyl)pyridin-2(1H)-one;
1-(4-fluorobenzyl)-5-(trifluoromethyl)pyridin-2(1H)-one;
1-(2-nitrobenzyl)-5-(trifluoromethyl)pyridin-2(1H)-one;
1-(2-aminobenzyl)-5-(trifluoromethyl)pyridin-2(1H)-one;
1-(3-chlorobenzyl)-5-(trifluoromethyl)pyridin-2(1H)-one;
1-(4-methoxybenzyl)-5-(trifluoromethyl)pyridin-2(1H)-one;
1-(2-fluorobenzyl)-5-(trifluoromethyl)pyridin-2(1H)-one;
1-(2-(2-hydroxyethylamino)benzyl)-5-(trifluoromethyl)pyridin-2(1H)-one;
1-(2-(2-(piperazin-1-yl)ethylaminof)benzyl)-5-(trifluoromethyl)pyridin-2(1H)-one;
1-(2-(2-(4-methylpiperazin-1-yl)ethylamino)benzyl)-5-(trifluoromethyl)pyridin-2(1H)-one;
1-(2-acetamide-benzyl)-5-(trifluoromethyl)pyridin-2(1H)-one;
1-(2-(2-morpholinoethylamino)benzyl)-5-(trifluoromethyl)pyridin-2(1H)-one;
1-(4-(2-(2-hydroxyethoxy)ethylamino)benzyl)-5-(trifluoromethyl)pyridin-2(1H)-one;

Also described is a synthetic method for compounds listed above, including: reacting 5-trifluoromethyl-2(1H)pyridone with substituted benzyl bromide, with DMSO as solvent, potassium carbonate as acid-binding agent to prepare a simple phenyl-substituted compound, shown in reaction formula I. The synthetic starting product trifluoromethyl pyridone is a commercial material. 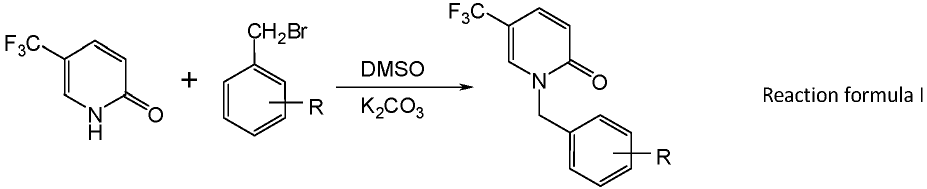 To prepare a simple amino-substituted compound, following reaction formula I to form nitro substituted derivatives; reducing the nitro substitute by iron powder in the presence of hydrochloric acid and preparing target products according to different compounds, shown in reaction formula II. 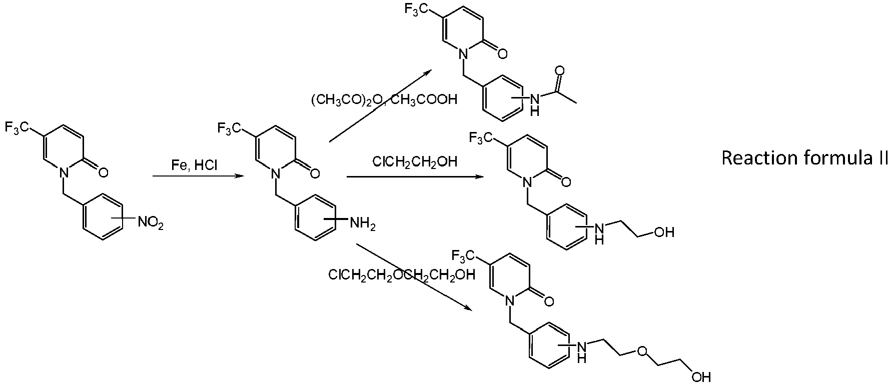 A compound, in which an amino group is bonded to a heterocyclic ring through an aliphatic side chain, is prepared including the steps of: preparing amino-substitute; and then reacting with heterocyclic compound with haloalkyl side chains, with DMF as solvent, potassium carbonate as acid-binding agent and sodium iodide as catalyst, shown in the reaction formula III. 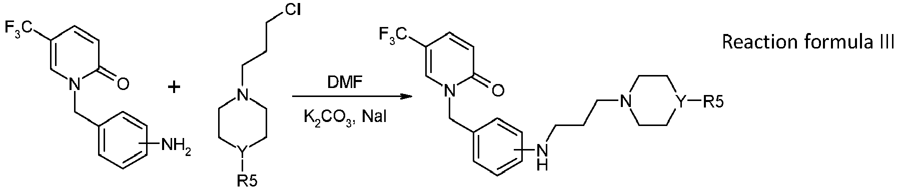 or, the target product is prepared by reacting hydroxyethyl amino substitute prepared a ccording to reaction formula II with thionyl chloride to produce chloroethyl amino sub stitute; and then reacting with the heterocyclic compound, shown in reaction formula I V.

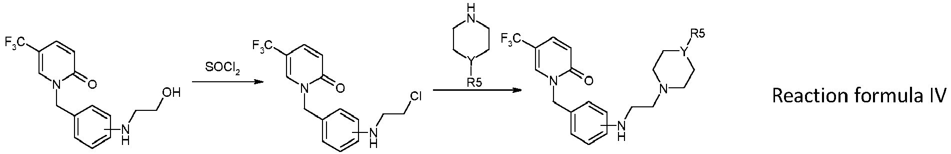

The above-mentioned compound is used for preparing a broad-spectrum medicament for fibrosis.
In the invention, based on the prior art, a substituted amino group is introduced onto the benzene ring at 1-position of pyridone; a hydrophilic group such as hydroxyl group and heterocyclic ring is introduced onto the amino group through an alkyl chain, thus obtaining a class of new pyridone compounds and salts thereof. The activity of the compounds is greatly enhanced.
The applicant finds that the produced compounds have relatively higher effects than the conventional pyridone compound by modifying the phenyl group by the substituted amino group on the basis of 1-phenyl-5-trifluoromethyl-pyridone; simultaneously the compounds including heterocyclic rings could be produced into various salts which are beneficial to being prepared into various liquid formulations.

### BRIEF DESCRIPTION OF DRAWINGS

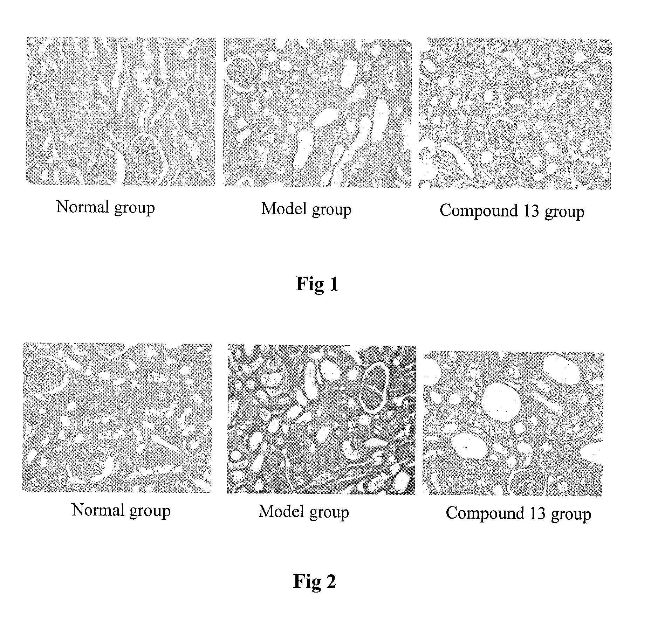

Figure 1 HE staining for renal pathology in embodiment 28 (×200)
Figure 2 Masson staining for renal pathology in embodiment 28 (×200)

### DETAILED DESCRIPTION OF PREFERRED EMBODIMENTS

### Example 1

### Preparation of 1-(4-nitrobenzyl)-5-(trifluoromethyl)pyridin-2(1H)-one

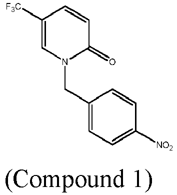

The preparation of 1-(4-nitrobenzyl)-5-(trifluoromethyl)pyridin-2(1H)-one includes steps of: adding 8.2g (0.050mol) of 5-(trifluoromethyl)pyridin-2(1H)-one in 100ml of DMSO for dissolving; adding 16.2g (0.075mol) of 1-(bromomethyl)-4-nitrobenzene, 11.0g (0.0 80mol) of potassium carbonate and allowing the resulting system to react at 85°C for 4 hours under stirring; after reaction, cooling to 40°C; adding 100ml of 12% ammonia solution; separating out a great amount of precipitate; filtering; dissolving the filter re sidue with ethyl acetate; decolorizing by active carbon; filtering; drying the filtrate by anhydrous sodium sulfate overnight; filtering out sodium sulfate; reclaiming part of s olvent to form crystals; filtering to obtain the product of 1-(4-nitrobenzyl)-5-(trifluorom ethyl)pyridin-2(1H)-one. The product is brown solid of 11.4g. m.p.:121∼123°C; EI-MS (m/z):288[M]⁺;1H-NMR(CDCl₃,300MHz) δppm: 5.240(s,2H,-CH₂-),6.694∼6.726(d1H,J=9. 6Hz,Ar-H),7.466∼7.482(d,2H,J=4.8Hz,Ar-H),7.495(s,1H,Ar-H)7.514∼7.522(d,1H,J=2.4Hz,A r-H),7.705(s,1H,Ar-H),8.222∼8.251(d,1H,J=8.7Hz,Ar-H).

### Example 2

### Preparation of 1-(4-amino-benzyl)-5-(trifluoromethyl)pyridin-2(1H)-one

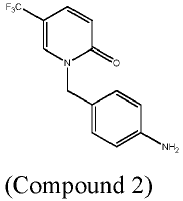

The preparation of 1-(4-amino-benzyl)-5-(trifluoromethyl)pyridin-2(1H)-one includes step s of: heating 11.4g (0.037mol) of 1-(4-nitrobenzyl)-5-(trifluoromethyl)pyridin-2(1H)-one, 200mL of 50% ethanol and 6.28g (0.112mol) of reductive iron powder to reflux; slo wly adding 0.42mL (0.004mol) of concentrated HCl in dropwise way (dropping after d ilution by 5mL of 50% ethanol); refluxing for 4 hours under stirring; after reaction, re gulating pH value to 10 by 15% KOH ethanol solution; filtering; washing the filter re sidues by 95% ethanol (2*10mL); extracting by ethyl acetate (50mL*3) after evaporati ng ethanol from the filtrate; drying the organic phase by anhydrous sodium sulfate ov ernight; filtering; and evaporating filtrate to obtain the product of 1-(4-amino-benzyl)-5 -(trifluoromethyl)pyridin-2(1H)-one. The product is brown solid powders of 9.9g. m.p.: 97∼98°C. ESI-MS(m/z): 291[M+Na]+.1H-NMR(CDCl₃,300MHz)δppm: 4.255(br,2H,-NH 2),5.023(s,2H,-CH₂-),6.629∼6.661(d,1H,J=5 9.6Hz,Ar-H),6.713∼6.740(d,2H,J=8.1Hz,Ar-H), 7.13∼7.164(d,2H,J=8.1Hz,Ar-H),7.393∼7.433(dd,1H,J=2.4Hz,9.6Hz,Ar-H),7.627(s,1H,Ar-H).

### Example 3

### Preparation of 1-(4-((3-morpholinylpropyl)amino)benzyl)-5-(trifluoromethyl)pyridin-2(1H)-one

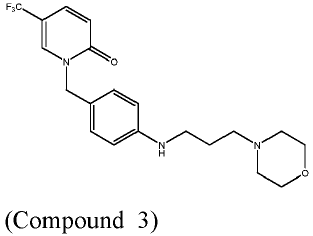

The preparation of 1-(4-((3-morpholinylpropyl)amino)benzyl)-5-(trifluoromethyl)pyridin-2(1H)-one includes steps of: adding 20mL of N,N-dimethylformamide to dissolve 2.01g (0.0075mol) of 1-(4-amino-benzyl)-5-(trifluoromethyl)pyridin-2(1H)-one; adding 0.69g(0.005mol) of potassium carbonate, 0.42g(0.0025mol) of 1-(3-chloro)propyl-morpholine and a catalytic amount of sodium iodide and allowing the resulting system to react at 130°C for 48 hours under stirring; filtering, evaporating filtrate to dryness; and separating residues by chromatography with eluent of petroleum ether and ethyl acetate with proportion of 1:1 (2% triethylamine) to obtain yellow oil of 0.5g. ESI-MS(m/z):396[M+H]+; H-NMR(CDCl₃,300MHz) δppm: 1.662∼1.750(m,2H,-CH₂-), 2.398∼2.569(m,6H,-CH₂-),3.103∼3.143(t,2H,-CH₂-),3.665∼3.756(t,4H,-CH₂-),4.780(br,1H,-N H-),4.934(s,2H,-CH₂-),6.438∼ .607(m,3H,Ar-H),7.065∼7.092(2H,Ar-H),7.314∼7.373(1H,Ar-H),7.553(s,1H,Ar-H).

### Example 4

### Preparation of 1-(4-(((3-piperidin-1-yl)propyl)amino)benzyl)-5-(trifluoromethyl)pyridin-2(1 H)-one

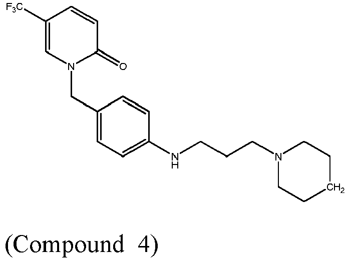

The preparation of 1-(4-(((3-piperidin-1-yl)propyl)amino)benzyl)-5-(trifluoromethyl)pyridin-2(1H)-one incudes steps of: adding 12mL of acetonitrile to dissolve 1.28g(0.0048mol) of 1-(4-amino-benzyl)-5-(trifluoromethyl)pyridin-2(1H)-one; adding 1.10g(0.008mol) of potassium carbonate, 0.64g(0.004mol) of 1-(3-chloro)propylpiperidine and a catalytic amount of sodium iodide and heating the resulting system to reflux for 48 hours under stirring; filtering, evaporating filtrate to dryness; and separating residues by chromatography with eluent of petroleum ether and ethyl acetate with proportion of 1:2 (1% triethylamine) to obtain off-white of 0.22g. m.p.:168∼170°C. ESI-MS(m/z): 394[+H]+. 1H-NMR(CDCl₃,300MHz)δppm: 1.582 (br,2H,-CH₂-), 1.859∼1.875 (m,4H, -CH₂-), 2.009∼2.071 (m,4H,-CH₂-) 2.806∼2.851 (t,6H,-CH₂-), 3.247∼3.286(t,2H,-CH₂-), 4.252(br,1H,-NH-),5.002(s,2H,-CH₂-),6.581∼6.609(d,2H,J=8.4Hz,Ar-H),6.620∼6.653(d,1H,J =9.9Hz,Ar-H),7.126∼7.154(d,2H,J=8.4Hz,Ar-H),7.385∼7.424(dd, 1H,J=2.1Hz,9.6Hz,Ar-H),7. 553(s,1H,Ar-H).

### Example 5

### Preparation of 1-(4-((3-(4-methyl-piperazin-1-yl)propyl)amino)benzyl)-5-(trifluoromethyl)p yridin-2(1H)-one

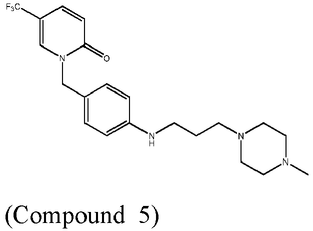

The preparation of 1-(4-((3-(4-methyl-piperazin-1-yl)propyl)amino)benzyl)-5-(trifluoromethyl)pyridin-2(1H)-on e incudes steps of: adding 3mL of ethanol to dissolve 0.402g(0.0015mol) of 1-(4-amino-benzyl)-5-(trifluoromethyl)pyridin-2(1H)-one; adding 0.088g(0.0005mol) of 1-(3-chloropropyl)-4-methylpiperazine and feeding a catalytic amount of potassium iodide; carrying out microwave reaction at 110°C; after reaction, filtering; evaporating filtrate to dryness; and separating residue by column chromatography with eluent of petroleum ether and ethyl acetate with proportion of 1:2 (1% triethylamine) to obtain yellow oil of 0.13g. ESI-MS(m/z): 409[M+H]+. 1H-NMR(CDCl₃,300MHz)δppm: 1.696∼1.802(m,2H,-CH₂-), 2.264(s,3H,-CH₃),2.427∼2.470(m,10H-CH₂-),3.094∼3.136(t,2H,-CH₂-),4.936(s,2H-CH₂-),6.4 88∼6.516(d,2H,J=8.4Hz,Ar-H),6.552∼6.583(d,1HJ=9.3Hz,Ar-H),7.061∼7.091(d,2H,J=9.0Hz, Ar-H),7.312∼7.352(dd,1H,J=2.4Hz,9.6Hz,Ar-H),7.551(s,1H,Ar-H).

### Example 6

### Preparation of 1-(4-((2-hydroxyethyl)amino)benzyl)-5-(trifluoromethyl)pyridin-2(1H)-one

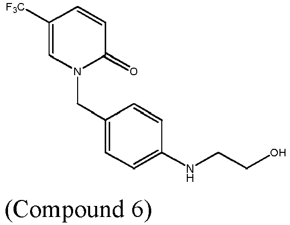

The preparation of 1-(4-((2-hydroxyethyl)amino)benzyl)-5-(trifluoromethyl)pyridin-2(1H)-one includes steps of: adding 100mL of n-butanol to dissolve 8.4g(0.03mol) of 1-(4-amino-benzyl)-5-(trifluoromethyl)pyridin-2(1H)-one; adding 4.1g(0.03mol) of potassium carbonate and 5.6g(0.06mol) of chloroethanol and allowing the resulting system to react at 130°C for 12 hours under stirring; filtering; evaporating filtrate to dryness; and separating residue by column chromatography with eluent of petroleum ether and ethyl acetate with proportion of 1:1 to obtain off-white solid of 2.0g. m.p.: 97∼98°C. ESI-MS(m/z): 335[M+Na]+.1H-NMR(CDCl₃,300MHz)δppm:3.293∼3.28(t,2H,-CH₂-),3.829∼3.864(t,2H,-C H₂-),5.015(s,2H,-CH₂-),6.623∼6.652(d,2H,J=8.7Hz,Ar-H),7.151∼7.179(d,2H,J=8.4Hz,Ar-H), 7.383∼7.424(dd,1H,J=2.7Hz,9.6Hz,Ar-H),7.446∼7.4588(dd,1H,J=2.7Hz,8.1Hz,Ar-H),7.621(s ,1H,Ar-H).

### Example 7

### Preparation of 1-(4-((2-(piperidyl-1-yl)ethyl)amino)benzyl)-5-(trifluoromethyl)pyridin-2(1H) -one

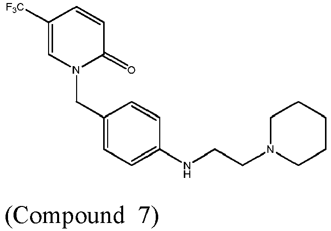

A Preparation of 1-(4-((2-chloroethyl)amino)benzyl)-5-(trifluoromethyl)pyridin-2(1H)-one The preparation of 1-(4-((2-chloroethyl)amino)benzyl)-5-(trifluoromethyl)pyridin-2(1H)-one includes steps of: dissolving 2.9mmol of 1-(4-((2-hydroxyethyl)amino)benzyl)-5-(trifluoromethyl)pyridin-2(1H)-one by 30ml of dichloromethane; adding 0.22ml of sulfurous dichloride AND 0.44ml of triethylamine; allowing the resulting system to react at room temperature for 12h under stirring; and separating residue by column chromatography with eluent of petroleum ether and ethyl acetate with proportion of 3:1 to obtain white solid of 0.24g.EI-MS(m/z):330[M]+.1H-NMR(CDCl₃,300MHz)δppm:3.485∼3.525(t,2H,-CH₂-),3.689 ∼3.728(t,2H,-CH₂-),4.181(br,1H,-NH-),5.020(s,2H,-CH₂-),6.612∼6.656(m,3H,Ar-H),7.167∼7. 195(d,2H,J=8.4Hz,Ar-H),7.385∼7.426(dd,1H,J=2.7Hz,9.6Hz,Ar-H),7.623(s, 1H,Ar-H).

### B Preparation of 1-(4-((2-(piperidyl-1-yl)ethyl)amino)benzyl)-5-(trifluoromethyl)pyridin-2(1H)-one

The preparation of 1-(4-((2-(piperidyl-1-yl)ethyl)amino)benzyl)-5-(trifluoromethyl)pyridin-2(1H)-one includes steps of: dissolving 0.24g(0.7mmol) of 1-(4-((2-chloroethyl)amino)benzyl)-5-(trifluoromethyl)pyridin-2(1H)-one in 30mL of acetonitrile; adding 0.37g(4.2mmol) of piperidine; carrying out refluxing reaction for 27 hours; filtering; evaporating filtrate to dryness; and separating by column chromatography with eluent of ethyl acetate and methanol with proportion of 10:1 to obtain yellow solid of 0.27g. m.p.: 83.6∼85.5°C.EI-MS(m/z): 379[M]+ .1H-NMR(CDCl₃,300MHz)δppm: 1.672(s,2H,-CH₂-),1.872(s,4H,-CH₂-),2.817∼3.112(br,6H,-CH₂-),3.542(s,2H,-CH₂-.),5.012(s, 2H,-CH₂-),5.174(br,1H,-NH-),6.618∼6.649(d,1H,J=9.3Hz,Ar-H),6.698∼6.726(d,2H,J=8.4Hz, Ar-H),7.152∼7.181 (d,1H,J=8.7Hz,Ar-H),7.386∼7.427(dd,1H,J=2.7Hz,9.6Hz,Ar-H),7.627(s,1 H,Ar-H).

### Example 8

### Preparation of 1-(4-((2-morpholinylethyl)amino)benzyl)-5-(trifluoromethyl)pyridin-2(1H)-o ne

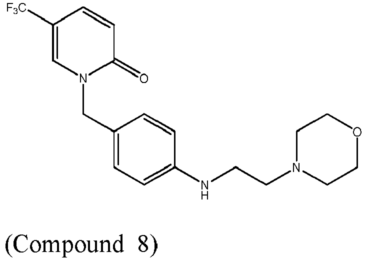

The preparation of 1-(4-((2-morpholinylethyl)amino)benzyl)-5-(trifluoromethyl)pyridin-2(1H)-one includes steps of: dissolving 0.26g(0.79mmol) of 1-(4-((2-chloroethyl)amino)benzyl)-5-(trifluoromethyl)pyridin-2(1H)-one in 30mL of acetonitrile; adding 0.41g(4.7mmol) of morpholine; carrying out refluxing reaction for 46 hours; filtering; evaporating filtrate to dryness; and separating by column chromatography with eluent of ethyl acetate and methanol with proportion of 10:1 1 to obtain brown oil of 0.29g. EI-MS(m/z):381[M]⁺.¹H-NMR(CDCl₃,300MHz)δppm:2.471∼2.484(br,4H,-CH₂-),2.614∼2.65 3(t,2H,-CH₂-),3.142∼3.181(t,2H,-CH₂-),3.704∼3.735(t,4H,-CH₂-),4.439(br,1H,-NH-), 5.012(s, 2H,-CH₂-),6.597∼6.650(m,3H,Ar-H),7.150∼7.178(d,2H,Ar-H),7.137∼7.417(dd,1H,J=2.7Hz,9. 6Hz,Ar-H),7.620(s, 1H,Ar-H).

### Example 9

### Preparation of 1-(4-((2-(4-methyl-piperazin-1-yl)ethyl)amino)benzyl)-5-(trifluoromethyl)pyr idin-2(1H)-one

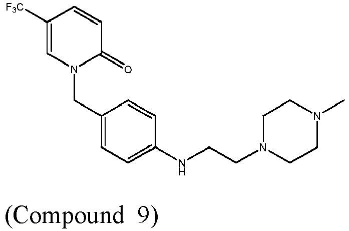

The preparation of 1-(4-((2-(4-methyl-piperazin-1-yl)ethyl)amino)benzyl)-5-(trifluoromethy 1)pyridin-2(1H)-one includes steps of: dissolving 0.33g(1.0mmol) of 1-(4-((2-chloroethyl) amino)benzyl)-5-(trifluoromethyl)pyridin-2(1H)-one in 30mL of acetonitrile; adding 0.60 g(6.0mmol) of N-methylpiperazine; carrying out refluxing reaction for 38 hours; filterin g; evaporating filtrate to dryness; and separating by column chromatography with elue nt of ethyl acetate and methanol with proportion of 10:1 to obtain brown oil of 0.31g. EI-MS(m/z): 394[M]⁺.¹H-NMR(CDCl₃,300MHz)δppm: 2.314(s,3H,-CH₃),2.512(br,8H,-C H₂-),2.622∼2.661(t,2H,-CH₂-),3.152(s,2H,-CH₂-),4.436(br,1H,-NH-),5.011(s,2H,-CH₂-),6.592 ∼6.650(t,3H,Ar-H),7.147∼7.175(d,2H,Ar-H),7.377∼7.417(dd,1H,J=2.4Hz,9.6Hz,Ar-H),7.622 (s,1H,Ar-H).

### Example 10

### Preparation 1-(4-((2-(piperazin-1-yl)ethyl)amino)benzyl)-5-(trifluoromethyl)pyridin-2(1H)-o ne

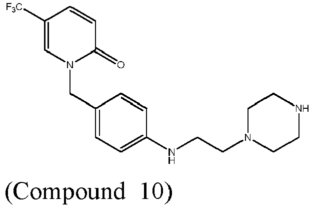

The preparation of 1-(4-((2-(piperazin-l-yl)ethyl)amino)benzyl)-5-(trifluoromethyl)pyridin-2(1H)-one includes steps of: dissolving 0.39g(1.2mmol) of 1-(4-((2-chloroethyl)amino)be nzyl)-5-(trifluoromethyl)pyridin-2(1H)-one in 30mL of acetonitrile; adding 0.82g(9.6mmo 1) of piperazine; carrying out refluxing reaction for 18 hours; filtering; evaporating filtr ate to dryness; and separating by column chromatography with eluent of ethyl acetate and methanol with proportion of 1:1 to obtain colorless oil of 0.37g. EI-MS(m/z): 380 [M]⁺.¹H-NMR(CDCl₃,300MHz)δppm: 2.445(br,4H,-CH₂-),2.593∼2.632(t,2H,-CH₂-),2.855∼ 2.915(t,4H,-CH₂-),3.132∼3.170(t,2H,-CH₂-),4.438(br,1H,-NH-),5.011(s,2H,-CH₂-),6.595∼6.6 50(t,3H,Ar-H),7.147∼7.175(d,2H,Ar-H),7.377∼7.417(dd,1H,J=2.4Hz,9.6Hz,Ar-H),7.625(s,1H, Ar-H).

### Example 11

### Preparation of 1-(4-((2-(4-(2-hydroxyethyl)piperazin-1-yl)ethyl)amino)benzyl)-5-(trifluorom ethyl)pyridin-2(1H)-one

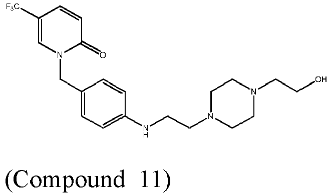

The preparation of 1-(4-((2-(4-(2-hydroxyethyl)piperazin-1-yl)ethyl)amino)benzyl)-5-(triflu oromethyl)pyridin-2(1H)-one includes steps of: dissolving 0.83g(2.5mmol) of 1-(4-((2-ch loroethyl)amino)benzyl)-5-(trifluoromethyl)pyridin-2(1H)-one and 2.6g(20mmol) of hydro xyethyl piperazine in 30mL of acetonitrile; adding an amount of sodium iodide; carryi ng out refluxing reaction for 29 hours; filtering; evaporating filtrate to dryness; and se parating by column chromatography with eluent of petroleum ether and ethyl acetate with proportion of 10:1 to obtain yellow oil of 0.60g. EI-MS(m/z): 424[M]⁺.¹H-NMR (CDCl₃,300MHz)δppm: 2.572∼2.268(m,12H,-CH₂-),3.151∼3.150(t,2H,-CH₂-),3.638∼3.673(t, 2H,-CH₂-),5.011(s,2H,-CH₂-),6.593∼6.649(t,3H,Ar-H),7.148∼7.176(d,2H,J=8.4Hz,Ar-H),7.37 7∼7.418(dd,1H,J=2.7Hz,9.6Hz,Ar-H),7.620(s,1H,Ar-H).

### Example 12

### Preparation of 1-(4-((2-(4-methyl-piperazin-1-yl)ethyl)amino)benzyl)-5-(trifluoromethyl)pyr idin-2(1H)-one dihydrochloride

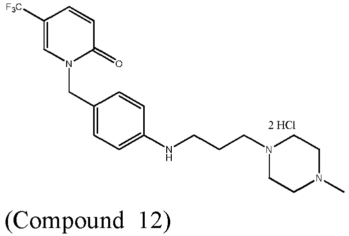

The preparation of 1-(4-((2-(4-methyl-piperazin-1-yl)ethyl)amino)benzyl)-5-(trifluoromethy 1)pyridin-2(1H)-one dihydrochloride includes steps of: dissolving 0.12g(1.1mmol) of 1-(4-((2-(4-methyl-piperazin-1-yl)ethyl)amino)benzyl)-5-(trifluoromethyl)pyridin-2(1H)-one b y 20ml of ethanol; adding 0.075mL of hydrochloric acid; mixing for reaction for 40m in under stirring; evaporating solvent to dryness to obtain 1-(2-chloro-4-(((3-piperidin-1-yl)propyl)amino)phenyl)-5-(trifluoromethyl)pyridin-2(1H)-one dihydrochloride which is ye llow solid of 0.09g. EI-MS(m/z): 394[M]⁺.¹H-NMR(D₂O)δppm: 2.961(s,3H,-CH₃),3.182∼ 3.236(t,2H,-CH₂-),3.299(s,2H,-H),3.389∼3.438(t,2H,-CH₂-),3.565(br,8H,-CH=),5.216(s,2H,-CH₂-),6.700∼6.731(d,1H,Ar-H),7.229∼7.257(d,2H,J=8.4Hz,Ar-H),7.340∼7.368(d,2H,J=8.4Hz, Ar-H),7.792∼7.823(d,1H,J=9.3Hz,Ar-H),8.275(s,1H,Ar-H).

### Example 13

### Preparation of 1-(4-acetamide-benzyl)-5-(trifluoromethyl)pyridin-2(1H)-one;

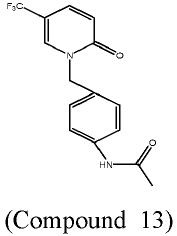

The preparation of 1-(4-acetamide-benzyl)-5-(trifluoromethyl)pyridin-2(1H)-one includes steps of: dissolving 1.1mol of acetic anhydride and 0.268g(lmmol) of 1-(4-amino-benz yl)-5-(trifluoromethyl)pyridin-2(1H)-one in 20ml of acetic acid; carrying out refluxing r eaction for 2 hours; adding 20ml of water and extracting by ethyl ether (2*20mL); w ashing the organic phase with 15% sodium bicarbonate solution and drying the organi c phase by anhydrous sodium sulfate; filtering; and evaporating filtrate; and separating residue by column chromatography with eluent of chloroform and methanol with prop ortion of 50:1 to obtain white solid of 0.20g. m.p.: 225.3∼227.2°C.ESI-MS(m/z): 333 [+Na]⁺.¹H-NMR(DMSO)δppm: 3.513(s,3H.-CH₃),5.098(s,2H,-CH₂-),6.566∼6.598(d,1H,J=9. 6Hz,Ar-H),7.269∼7.296(d,2H,J=8.1Hz,Ar-H),7.524∼7.551(d,2H,J=8.1Hz,Ar-H),7.678∼7.704 (d,1H,J=8.4Hz,Ar-H),8.5124(s,1H,Ar-H),10.009(s,1H,-NH).

### Example 14

### Preparation of 1-(2,6-dichlorobenzyl)-5-(trifluoromethyl)pyridin-2(1H)-one

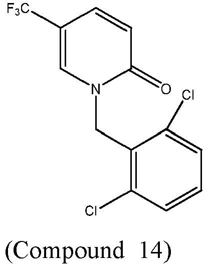

The preparation of 1-(2,6-dichlorobenzyl)-5-(trifluoromethyl)pyridin-2(1H)-one includes steps of: dissolving 0.49g(3.0mmol) of 5-(trifluoromethyl)pyridin-2(1H)-one in 30ml of DMF; adding 0.5g(3.6mmol) of sodium carbonate and 0.88g(4.5mmol) 1,3-dichloro-2-(chloromethyl)benzene; carrying out refluxing reaction for 3 hours; filtering; evaporating filtrate; and separating residue by column chromatography with eluent of petroleum ether to obtain the product 1-(2,6-dichlorobenzyl)-5-(trifluoromethyl)pyridin-2(1H)-one as light-yellow solid of 0.72g. m.p.: 74.0∼76.0°C.EI-MS(m/z): 321[M-1]⁺.¹H-NMR(CDCl₃,300MHz)δppm: 5.442(s,2H,-CH₂-),6.665∼6.697(d,1H,J=9.6Hz,Ar-H),7.266(1H,Ar-H),7.318∼7.431(dd,1H,J= 7.2Hz,9.3Hz,Ar-H),7.413∼7.460(m,3H,Ar-H).

### Example 15

### Preparation of 1-(4-fluorobenzyl)-5-(trifluoromethyl)pyridin-2(1H)-one

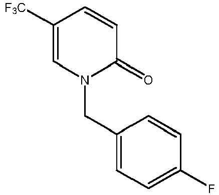

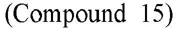

The preparation of 1-(4-fluorobenzyl)-5-(trifluoromethyl)pyridin-2(1H)-one includes steps of: dissolving 0.49g(3.0mmol) of 5-(trifluoromethyl)pyridin-2(1H)-one in 30ml of DMF; adding 0.5g(3.6mmol) of sodium carbonate and 0.85g(4.5mmol) 1-(bromomethyl)-4-fluorobenzene; carrying out refluxing reaction for 3 hours; filtering; evaporating filtrate; and separating residue by column chromatography with eluent of petroleum ether to obtain the product 1-(4-fluorobenzyl)-5-(trifluoromethyl)pyridin-2(1H)-one as white solid of 0.72g. m.p.: 70.1∼72.0 °C. ESI-MS(m/z): 294[M+Na]⁺. ¹H-NMR(CDCl₃,300MHz)δppm: 5.112(s,2H,-CH₂-),6.658∼6.690(d,1H,J=9.6Hz,Ar-H),7.035∼7.093(m,2H,Ar-H),7.299∼7.345( m,2H,Ar-H),7.423∼7.464(dd,1H,J=2.7Hz,9.6Hz,Ar-H),7.649(s,1H,Ar-H).

### Example 16

### Preparation of 1-(2-nitrobenzyl)-5-(trifluoromethyl)pyridin-2(1H)-one

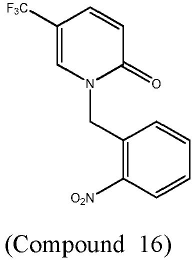

The preparation of 1-(2-nitrobenzyl)-5-(trifluoromethyl)pyridin-2(1H)-one includes steps of: dissolving 12.3g(0.075mol) of 5-(trifluoromethyl)pyridin-2(1H)-one in 200ml of DMF; adding 16.6g(0.12mol) of sodium carbonate and 24.5g(0.113mol) 1-(bromomethyl)-2-nitrobenzene; carrying out refluxing reaction for 4 hours; cooling to 40 °C; adding 40ml of 15% ammonia solution; extracting by ethyl acetate (50mL*3); decolorizing by active carbon; drying by anhydrous sodium sulfate; filtering; evaporating filtrate; and separating residue by column chromatography to obtain the product 1-(2-nitrobenzyl)-5-(trifluoromethyl)pyridin-2(1H)-one as white solid of 12.2g. m.p.: 100.0∼102.0 °C. EI-MS(m/z): 298[M] ⁺.¹H-NMR(CDCl₃,300MHz)δppm: 5.547(s,2H,-CH₂-),6.698∼6.730(d,1H,J=9.6Hz,Ar-H),7.179∼7.204(d,1H,J=7.5Hz,Ar-H),7.494 ∼7.553(m,2H,Ar-H),7.600∼7.655(m,1H,Ar-H),7.794(s, 1H,Ar-H),8.136∼8.168(dd,1H,J=1.5Hz ,8.4Hz,Ar-H).

### Example 17

### Preparation of 1-(2-aminobenzyl)-5-(trifluoromethyl)pyridin-2(1H)-one

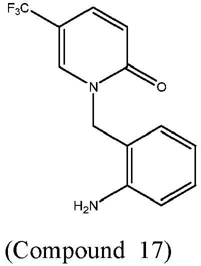

The preparation of 1-(2-aminobenzyl)-5-(trifluoromethyl)pyridin-2(1H)-one includes steps of: heating 11.7g (0.039mol) of 1-(2-nitrobenzyl)-5-(trifluoromethyl)pyridin-2(1H)-one, 400mL of 50% ethanol and 6.6g (0.118mol) of reductive iron powder to reflux; slowly adding 5mL of concentrated HCl in 50% ethanol in dropwise way; refluxing for 2 hours under stirring; after reaction, cooling to 50°C; regulating pH value to 8 by 15% KOH ethanol solution; filtering; extracting by ethyl acetate (100+100+50mL) after evaporating ethanol from the filtrate to half volume; drying the organic phase by anhydrous sodium sulfate; filtering; and evaporating filtrate to the volume of 20mL to obtain the crystal product of 1-(2-aminobenzyl)-5-(trifluoromethyl)pyridin-2(1H)-one. The product is light-yellow solid. m.p.:83.4∼85.3°C.EI-MS(m/z):268[M]⁺.¹H-NMR(CDCl₃,300MHz):δppm5.098(s,2H,-CH₂-),6 .656∼6.687(d,1H,J=9.3Hz,Ar-H),6.724∼6.749(d,1H,J=7.5Hz,Ar-H),6.770∼6.795(d,1H,J=7.5 Hz,Ar-H),7.164∼7.208(m,2H,Ar-H),7.431∼7.472(dd,1H,J=2.7Hz,9.6Hz,Ar-H),7.755(s,1H,Ar-H).

### Example 18

### Preparation of 1-(3-chlorobenzyl)-5-(trifluoromethyl)pyridin-2(1H)-one

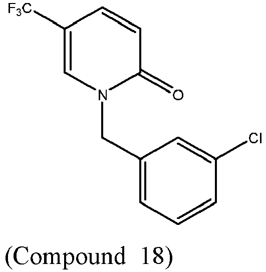

The preparation of 1-(3-chlorobenzyl)-5-(trifluoromethyl)pyridin-2(1H)-one includes steps of: dissolving 0.49g(3.0mmol) of 5-(trifluoromethyl)pyridin-2(1H)-one in 20ml of DMF; adding 0.66g(4.8mmol) of sodium carbonate and 0.93g(4.5mmol) 1-(bromomethyl)-3-chlorobenzene; carrying out refluxing reaction for 3 hours; adding 40ml of 15% ammonia solution; extracting by ethyl acetate (30+20+20mL); drying by anhydrous sodium sulfate; filtering; evaporating filtrate; and separating residue by column chromatography with eluent of petroleum ether and ethyl acetate with proportion of 6:1 to obtain the product 1-(3-chlorobenzyl)-5-(trifluoromethyl)pyridin-2(1H)-one as colorless oil of 0.70g.EI-MS(m/z):287[M]+.¹H-NMR(CDCl₃,300MHz)δppm:5.098(s,2H,-CH₂-),6.656∼6.687( d,1H,J=9.3Hz,Ar-H),7.186∼7.210(m,1H,Ar-H),7.307∼7.324(m,3H,Ar-H),7.442∼7.482(dd,1H, J=2.4Hz,9.6Hz,Ar-H),7.650(s,1H,Ar-H).

### Example 19

### Preparation of 1-(4-methoxybenzyl)-5-(trifluoromethyl)pyridin-2(1H)-one

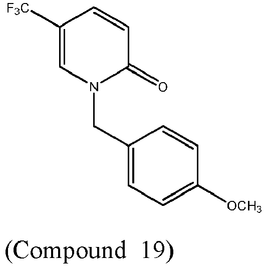

The preparation of 1-(4-methoxybenzyl)-5-(trifluoromethyl)pyridin-2(1H)-one includes steps of: dissolving 0.50g(3.0mmol) of 5-(trifluoromethyl)pyridin-2(1H)-one in 20ml of DMF; adding 0.66g(4.8mmol) of sodium carbonate and 0.91g(4.5mmol) 1-(bromomethyl)-4-methoxybenzene; carrying out refluxing reaction for 3 hours; adding 40ml of 15% ammonia solution; extracting by ethyl acetate (30+20+20mL); drying by anhydrous sodium sulfate; filtering; evaporating filtrate; and separating residue by column chromatography with eluent of petroleum ether and ethyl acetate with proportion of 8:1 to obtain the product 1-(4-methoxybenzyl)-5-(trifluoromethyl)pyridin-2(1H)-one as white solid of 0.79g. m.p.: 84.2∼86.1°C. EI-MS(m/z): 283[M]⁺.¹H-NMR(CDCl₃,300MHz):δppm: 3.806(s,3H,-CH₃),5.077(s,2H,-CH₂-),6.638∼6.670(d,1H,J=9.6Hz,Ar-H),6.885∼6.891(d,1H,J= 1.8Hz,Ar-H),.907∼6.914(d,1H,J=2.1Hz,Ar-H),7.259∼7.287(m,3H,Ar-H),7.398∼7.439(dd,1H,J =2.7Hz,9.6Hz,Ar-H),7.633(s,1H,Ar-H).

### Example 20

### Preparation of 1-(2-fluorobenzyl)-5-(trifluoromethyl)pyridin-2(1H)-one

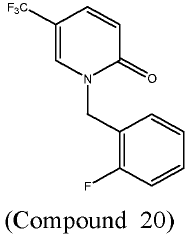

The preparation of 1-(3-chlorobenzyl)-5-(trifluoromethyl)pyridin-2(1H)-one includes steps of: dissolving 0.49g(3.0mmol) of 5-(trifluoromethyl)pyridin-2(1H)-one in 20ml of DMF; adding 0.66g(4.8mmol) of sodium carbonate and 0.85g(4.5mmol) 1-(bromomethyl)-2-fluorobenzene; carrying out refluxing reaction for 3 hours; adding 40ml of 15% ammonia solution; extracting by ethyl acetate (30+20+20mL); drying by anhydrous sodium sulfate; filtering; evaporating filtrate; and separating residue by column chromatography with eluent of petroleum ether and ethyl acetate with proportion of 6:1 to obtain the product 1-(2-fluorobenzyl)-5-(trifluoromethyl)pyridin-2(1H)-one as colorless oil of 0.65g.EI-MS(m/z):271[M]⁺.¹H-NMR(CDCl₃,300MHz):δppm:5.176(s,2H,-CH₂-),6.623∼6.655 (d,1H,J=9.6Hz,Ar-H),7.074∼7.178(m,2H,Ar-H),7.301∼7.377(m,1H,Ar-H),7.410∼7.505(m,2H ,Ar-H),7.795(s,1H,Ar-H).

### Example 21

### Preparation of 1-(2-(2-hydroxyethylamino)benzyl)-5-(trifluoromethyl)pyridin-2(1H)-one

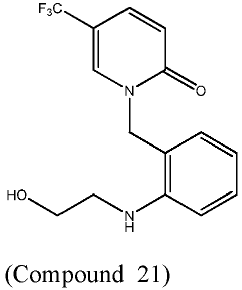

The preparation of 1-(2-(2-hydroxyethylamino)benzyl)-5-(trifluoromethyl)pyridin-2(1H)-one includes steps of: adding 100mL of n-butanol to dissolve 9.0g(0.034mol) of 1-(2-amino-benzyl)-5-(trifluoromethyl)pyridin-2(1H)-one; adding 4.69g(0.034mol) of potassium carbonate and 4.1g(0.05mol) of chloroethanol and allowing the resulting system to react at 130°C for 18 hours under stirring; filtering; evaporating filtrate to dryness; and separating residue by column chromatography with eluent of petroleum ether and ethyl acetate with proportion of 5:1 to obtain 1-(2-(2-hydroxyethylamino)benzyl)-5-(trifluoromethyl)pyridin-2(1H)-one as white solid of 0.5g. m.p.: 114.0∼115.0°C.EI-MS(m/z):312[M] ⁺.¹H-NMR(CDCl₃,300MHz):δppm: 2.822(s,1H,-OH),3.228∼3.261(t,2H,-CH₂-),3.878(s,2H,-CH₂-),5.109(s,2H,-CH₂-),5.843(br,1H ,-NH-),6.620∼6.647(d,1H,Ar-H),6.686∼6.736(m,2H,Ar-H),7.206∼7.303(m,2H,Ar-H),7.458∼7 .499(dd,1H,J=2.7Hz,9.6Hz,Ar-H),7.802(s,1H,Ar-H).

### Example 22

### Preparation of 1-(2-(2-(piperazin-1-yl)ethylaminof)benzyl)-5-(trifluoromethyl)pyridin-2(1H)-one

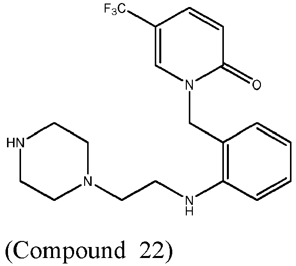

A Preparation of 1-(2-((2-chloroethyl)amino)benzyl)-5-(trifluoromethyl)pyridin-2(1H)-one The preparation of 1-(2-((2-chloroethyl)amino)benzyl)-5-(trifluoromethyl)pyridin-2(1H)-one includes steps of: dissolving 2.2g of 1-(2-((2-hydroxyethyl)amino)benzyl)-5-(trifluoromethyl)pyridin-2(1H)-one by 40ml of dichloromethane; adding 1.7g of sulfurous dichloride and 1.2g of triethylamine; allowing the resulting system to react at room temperature for 10 hours under stirring; and separating residue by column chromatography to obtain white solid of 1.5g. m.p.: 93.5∼95.0°C.EI-MS(m/z):330[M]⁺.¹H-NMR(CDCl₃,300MHz): δppm: 3.483∼3.543(t,2H,-CH₂-),3.628∼3.672(t,2H,-CH₂-),5.089(s,2H,-CH₂-),5.737∼5.753(1H,-NH-) ,6.618~6.757(m,3H,Ar-H),7.206∼7.235(dd,1H,J=1.2Hz,7.5Hz,Ar-H),7.250∼7.307(dd,1H,J=8. 1Hz,,9.0Hz,Ar-H),7.732(s,1H,Ar-H).
**B** Preparation of 1-(2-(2-(piperazin-1-yl)ethylaminof)benzyl)-5-(trifluoromethyl)pyridin-2(1H)-one The preparation of 1-(2-(2-(piperazin-1-yl)ethylaminof)benzyl)-5-(trifluoromethyl)pyridin-2 (1H)-one includes steps of: dissolving 0.33g(1mmol) of 1-(2-((2-chloroethyl)amino)benz yl)-5-(trifluoromethyl)pyridin-2(1H)-one in 20mL of acetonitrile; adding 0.53g(6mmol) o f anhydrous piperazine and a catalytic amount of sodium iodide; carrying out refluxing reaction for 20 hours; filtering; evaporating filtrate to dryness; and separating by colu mn chromatography with methanol to obtain yellow oil of 0.26g. EI-MS(m/z):380[M]⁺. ¹H-NMR(CDCl₃,300MHz): δppm: 2.434(s,4H,-CH₂-),2.575∼2.615(t,2H,-CH₂-),2.839∼2.853 (d,4H,-CH₂-),3.181∼3.200(d,2H,-CH₂-),5.153(s,2H,-CH₂-),5.275(s,1H,-NH-),6.636∼6.740(m, 3H,Ar-H),7.150∼7.174(d,1H,J=7.2Hz,Ar-H),7.283∼7.309(m, 1H,Ar-H),7.426∼7.458(d, 1H,J=9. 6Hz,Ar-H),7.632(s,1H,Ar-H).

### Example 23

### Preparation of 1-(2-(2-(4-methylpiperazin-1-yl)ethylamino)benzyl)-5-(trifluoromethyl)pyridi n-2(1H)-one

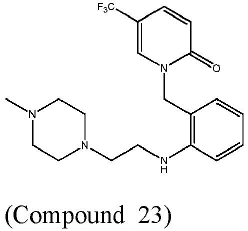

The preparation of 1-(2-(2-(4-methylpiperazin-1-yl)ethylamino)benzyl)-5-(trifluoromethyl) pyridin-2(1H)-one includes steps of: dissolving 0.33g(lmmol) of 1-(2-((2-chloroethyl)am ino)benzyl)-5-(trifluoromethyl)pyridin-2(1H)-one in 20mL of acetonitrile; adding 0.55g(5 mmol) of 1-methylpiperazine and a catalytic amount of sodium iodide; carrying out re fluxing reaction for 20 hours; filtering; evaporating filtrate to dryness; and separating by column chromatography with ethyl acetate (2% triethylamine) to obtain product 1-(2-(2-(4-methylpiperazin-1-yl)ethylamino)benzyl)-5-(trifluoromethyl)pyridin-2(1H)-one as y ellow oil of 0.28g. ESI-MS(m/z): 395[M+H]⁺.¹H-NMR(CDCl₃,300MHz): δppm: 2.308(s, 3H,-CH₃),2.526(br,8H, -CH₂-),2.603∼2.645(t,2H,-CH₂-),3.159∼3.216(m.2H,-CH₂-),5.059(s,2 H,-CH₂-),5.148(s,1H,-NH-),6.364∼6.742(m,3H,Ar-H),7.157∼7.175(d,1H,J=7.2Hz,Ar-H),7.28 4∼7.301(1H,Ar-H),7.423∼7.462(dd,1H,J=2.4Hz,9.3Hz,Ar-H),7.631(s,1H,Ar-H).

### Example 24

### Preparation of 1-(2-acetamide-benzyl)-5-(trifluoromethyl)pyridin-2(1H)-one

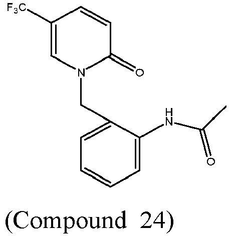

The preparation of 1-(2-acetamide-benzyl)-5-(trifluoromethyl)pyridin-2(1H)-one includes steps of: dissolving 1.1mol of acetic anhydride and 0.32g(1mmol) of 1-(2-amino-benzyl) -5-(trifluoromethyl)pyridin-2(1H)-one in 20ml of acetic acid; carrying out refluxing reac tion for 2 hours; adding 20ml of water and extracting by ethyl ether (2*20mL); washi ng the organic phase with 15% sodium bicarbonate solution and drying the organic ph ase by anhydrous sodium sulfate; filtering; and evaporating filtrate; and separating resi due by column chromatography with eluent of petroleum ether and ethyl acetate with proportion of 3:1 to obtain white solid of 0.20g. m.p.: 183.0∼185.0°C.ESI-MS(m/z): 3 33[+Na]⁺.¹H-NMR (CDCl₃,300MHz)δppm: 2.280(s,3H-CH₃),5.113(s,2H,-CH₂-),6.703∼6.73 5(d,1H,J=9.6Hz,Ar-H),7.114∼7.163(t,1H,Ar-H),7.338∼7.424(m.2H,Ar-H),7.16∼7.53(dd,1H, J=2.4Hz,9.3Hz,Ar-H),7.904(s,1H,Ar-H),8.143∼8.170(d,1H,J=8.1Hz,Ar-H),9.975(s,1H,-NH-).

### Example 25

### Preparation of 1-(2-(2-morpholinoethylamino)benzyl)-5-(trifluoromethyl)pyridin-2(1H)-one

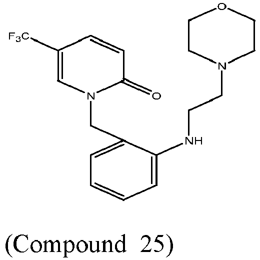

The preparation of 1-(2-(2-morpholinoethylamino)benzyl)-5-(trifluoromethyl)pyridin-2(1H) -one includes steps of: dissolving 0.26g(0.79mmol) of 1-(2-((2-chloroethyl)amino)benzyl) -5-(trifluoromethyl)pyridin-2(1H)-one in 20mL of acetonitrile; adding 1.0g(11.5mmol) of morpholine and a catalytic amount of sodium iodide; carrying out refluxing reaction f or 25 hours; filtering; evaporating filtrate to dryness; dissolving the residue with ethyl acetate and washing with water; drying the organic phase by anhydrous sodium sulfat e; and separating by column chromatography with eluent of petroleum ether and ethyl acetate with proportion of 1:1 to obtain product 1-(2-(2-morpholinoethylamino)benzyl)-5-(trifluoromethyl)pyridin-2(1H)-one as off-white of 0.18g. m.p.: 101.0∼103.0°C.EI-MS (m/z): 381[M]⁺.¹H-NMR(CDCl₃,300MHz): δppm: 2.472(s,4H,-CH₂-),2.616(s,2H, -CH₂-),3. 201(s,2H,-CH₂-),3.685(s,4H,-CH₂-),5.071(s,2H,-CH₂-),5.179(s,1H,-NH-),6.633∼6.746(m,3H, Ar-H),7.163∼7.187(d,1H,J=7.2Hz,Ar-H),7.285∼7.311(1H,Ar-H),7.423∼7.463(dd,1H,J=2.4Hz, 9.6Hz,Ar-H),7.643(s, 1H,Ar-H).

### Example 26

### Preparation of 1-(4-(2-(2-hydroxyethoxy)ethylamino)benzyl)-5-(trifluoromethyl)pyridin-2(1 H)-one

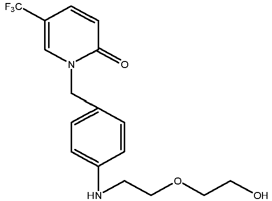

The preparation of 1-(4-(2-(2-hydroxyethoxy)ethylamino)benzyl)-5-(trifluoromethyl)pyridin-2(1H)-one includes steps of: dissolving 1-(4-((2-chloroethyl)amino)benzyl)-5-(trifluoromethyl)pyridin-2(1H)-one and 0.37g(3mmol) of chloroethoxy ethanol in 20mL of normal butanol; adding 0.28g(2mmol) of potassium carbonate and a catalytic amount of sodium iodide; carrying out refluxing reaction for 28 hours; after reaction, filtering; evaporating filtrate to dryness; and separating by column chromatography with eluent of petroleum ether and ethyl acetate with proportion of 2:1 1 to obtain yellow oily product of 0.22g. EI-MS(m/z): 356[M] ⁺.¹H-NMR(CDCl₃,300MHz)δppm:3.312∼3.346(t,2H,-CH₂-),3.589∼3.619(t,2H,-CH₂-),3.652∼ 3.776(t,4H,-CH₂-),5.019(s,2H,-CH₂-),6.623∼6.679(t,3H,Ar-H),7.154∼7.182(d,2H,Ar-H),7.384 ∼7.425(dd, 1H,J=2.7Hz,9.6Hz,Ar-H),7.619(s, 1H,Ar-H).

### Example 27 Inhibition test of compounds to NIH3T3 mechanocytes

An MTT method is used and comprises steps of: culturing cells in DMEM culture medium including 5% calf serum and preparing the cells into cell suspension of 3*10⁴/ml; inoculating in 96-pore plate according to 100 µl/pore; transferring new culture medium including compounds with different concentration, fluorofenidone and 1% calf serum after cells are adhered, wherein three repeated pores are provided for each concentration; respectively adding 100 µl of MTT solution in each pore after 48 hours and 72 hours of administrating (the culture medium is prepared into 5mg/ml and kept in dark after filtering), sucking out MTT after 4 hours; adding 150 µl of DMSO which is the dissolving liquid of MTT; after 10min and MTT is completely dissolved, measuring OD value by ELISA reader; calculating IC₅₀ values of fluorofenidone and measured compounds according to inhibition ratio; figuring out multiple of activities of measured compounds and fluorofenidone according to IC₅₀ values of fluorofenidone and measured compounds; and obtaining relative IC₅₀ value of measured compounds according to multiple and IC₅₀ value of fluorofenidone on a certain plate.

### Inhibition activity of measured compounds to NIH3T3 mechanocyte

| Measured compounds | 48hours | | 72hours | |
|---|---|---|---|---|
| | Relative IC₅₀ (mM) | Multiple | Relative IC₅₀ (mM) | Multiple |
| Fluorofenidone | 4.43 | ----- | 3.52 | ----- |
| Structure IV | 1.160 | 3.82 | 0.729 | 4.83 |
| Compound 1 | 0.552 | **8.03** | 0.470 | **7.50** |
| Compound 2 | 1,393 | **3.18** | 1.511 | **2.33** |
| Compound 3 | 0.424 | **10.46** | 0.229 | **15.39** |
| Compound 4 | 0.957 | **4.63** | 0.144 | **24.43** |
| Compound 5 | 0.141 | **31.39** | 0.098 | **35.83** |
| Compound 6 | 1.011 | **4.38** | 0,638 | **5.51** |
| Compound 7 | 4.179 | **1.06** | 0.340 | **10.36** |
| Compound 8 | 0.367 | **12.07** | 0.229 | **15.36** |
| Compound 9 | 0.181 | **24.53** | 0.120 | **29.38** |
| Compound 11 | 0.526 | **8.42** | 0.131 | **26.88** |
| Compound 13 | 0.128 | **34.55** | 0.068 | **52.14** |
| Compound 14 | 0.346 | **12.81** | 0.182 | **19.29** |
| Compound 15 | 1.078 | **4.11** | 1.093 | **3.22** |
| Compound 16 | 1.035 | **4.28** | 1.032 | **3.41** |
| Compound 17 | 1.808 | **2.45** | 1.271 | **2.77** |
| Compound 18 | 1.457 | **3.04** | 1.349 | **2.61** |
| Compound 19 | 0.301 | **14.72** | 0.384 | **9.16** |
| Compound 20 | 0.273 | **16.25** | 0.393 | **8.95** |
| Compound 21 | 0.664 | **6.67** | 0.587 | **6.00** |
| Compound 22 | 0.168 | **26.31** | 0.291 | **12.10** |
| Compound 25 | 0.368 | **12.04** | 0.264 | **13.33** |

| | | | | |
|---|---|---|---|---|
| Notes: multiple is IC₅₀ value of compounds to IC₅₀ value of fluorofenidone | | | | |

### Example 28

### Observation of treatment effect of compound 13 to rat unilateral ureteral obstruction renal fibrosis model

### Materials and methods

### 1. Experimental chemicals

The compound 13 is prepared according to the method provided by the invention.

### 2. Experimental animals

Nine male SD rats of 188-213g, coming from Hunan Slac Laobratory Animals Co., Ltd., are illuminated for 12 hours every day; feed is provided by Shanghai Slac Laobratory Animals Co., Ltd.; and drinking water is provided by Department of Laboratory Animal Science of Central South University.

### 3. Experimental methods

(1) **Randomization**: nine rats are divided into three groups at random, namely a normal group (n=3); a model group (n=3) and a treatment group (n=3) treated by compound 13 of 15mg/kg; three rats are in a hutch; and the experimental animals are adaptively fed for two days.
**(2) Unilateral ureteral obstruction modeling:**
   The unilateral ureteral obstruction modeling comprises steps of: lumbar-injecting each rat with 10% chloral hydrate according to 0.35m1/100g for anesthesia, fixing on a rat fixing plate; wetting the back skin by water, tightening the skin; unhairing by elbowed surgical scissors in a way closely attaching the skin; sterilizing drape in a conventional way; making an incision of 1.0cm in longitudinal direction at a junction of a position 1.0cm below left costal margin and 0.8cm next to median line of vertebral column; separating successive layers to expose left kidney and left ureter; tying off left ureter against lower pole of left kidney by a thread of 4.0 and another portion 1.0cm therebelow; isolating ureter between those two points; flushing abdominal cavity by gentamicin physiological saline solution; and stitching successive layers of retroperitoneal space and back skins after no leakage and hemorrhage.
**(3) Pharmacological intervention:** intragastric administration is carried out the day before modeling operation according to one time per day for 12 days; the method is detailed as follows:
   a) preparing 0.5% CMCNa solution by adding an amount of 0.9% physiological saline into CMCNa powder and preparing following groups of chemicals with 0.5% CMCNa solution as solvent.
   b) lavaging the normal group by 0.5% CMC-NA of 6ml/kg.d for one time per day.
   c) lavaging the model group by 0.5% CMC-NA of 6ml/kg.d for one time per day.
   d) lavaging the treatment group treated by compound 13 of 15mg/kg by 0.5% CMC-NA of 6ml/kg.d for one time per day.
**(4) Animal sacrifice and sample collection**
   Each group of rats is respectively lumbar-injected with 10% chloral hydrate (0.7-0.9m1/100g) on 11st day after operation for excessive anesthesia until sacrifice, renal tissues on the obstruction side is fixed by 4% formaldehyde, embedded by paraffin and prepared into 4 mum-thick slices for HE staining and Masson staining.
**(5) HE staining evaluation standard:**
   HE staining slices of renal tissues are successively observed in fives fields of view of renal tubulointerstitium on upper left side, upper right side, lower left side, lower right side and middle portion by a low power lens and are evaluated according to eight indexes of renal interstitium lesion: renal tubular epithelial cell vacuolar degeneration, renal tubular ectasia, renal tubular atrophy, red cell cast, protein cast, interstitial edema, interstitial fibrosis and interstitial inflammatory cell infiltration; an average value is calculated as the index of renal tubulointerstitial lesion of the sample; and the evaluation standard is based on the reference of Radford MG Jr, Donadio JV Jr, Bergstralh EJ, et al. Predicting renal outcome in IgA nephropathy. J Am Soc Nephrol, 1997, 8(2):199-207.
**(6) Masson staining evaluation standard**
   Masson staining slices of renal tissues are observed in 20 fields of vision for each sample at random under 400X light microscope; percent of blue-stain collagens in the fields of vision is calculated; an average value is determined after semi-quantitative evaluation: no positive staining, 0; <25%, 1; 25-50%, 2; 50-75%, 3; >75 %, 4; and the evaluation standard is based on references.

### 4. Statistical methods: analytical method of variance of single factor is adopted.

### Experimental Results

### 1. Pathological evaluation results of renal interstitium lesions through HE staining

**Table 1 comparison of indexes of renal tubulointerstitial lesions of obstruction kidneys of rats in groups**

| Group | Number | Score**(X̅±S)** |
|---|---|---|
| Normal group | 3 | 0.33±0.12 |
| Model group | 3 | 9.00±1.00^{★★★} |
| Compound 13 group | 3 | 7.00±0.35^{★★}** |

| | | |
|---|---|---|
| **Notes:** **comparison to normal group,** ^{★}<p0.05, ^{★★}p<0.01; ^{★★★}p<0.001; **comparison to model group,** *p<0.05, ** p<0.01, *** p<0.001; | | |

### 2. Pathological evaluation results of renal interstitium lesions through MASSON staining

**Table 2 evaluation results of renal interstitium collagens of left kidneys of rats in groups through MASSON staining**

| Group | Number | Score**(X̅±S)** |
|---|---|---|
| Normal group | 3 | 0.25±0.00 |
| Model group | 3 | 2.45±0.38^{★★★} |
| Compound 13 group | 3 | 1.52±0.16^{★★}** |

| | | |
|---|---|---|
| **Notes:** **comparison to normal group,** ^{★}p<0.05, ^{★★}p<0.01; ^{★★★}p<0.001; **comparison to model group,** *p<0.05, **p<0.01, ***p<0.001; | | |

### Conclusion:

The compound 13 of 15mg/kg can effectively treat renal fibrosis.

## Claims

1. 1-(substituted benzyl)-5-trifluoromethyl-2(1H)pyridone compounds, having a structural formula (XIII), 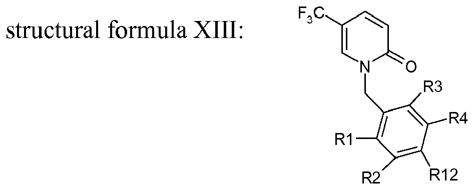 wherein R1-R4, R12 are selected from H, NO₂, hydroxyl, amino, halogen atom, C₁-C₆ alkoxyl, NR¹⁰R¹¹, OR¹³, C(O)R¹⁴, O-C(O)R¹⁴, C₁-C₆ alkyl, C₁-C₆ haloalkyl, C²-C⁶ alkenyl; wherein R1∼R4, R12 are not simultaneously H, R¹⁴ is selected from C₁-C₆ alkyl, R¹³ is selected from hydroxyalkyl or alkoxyalkyl; wherein in NR¹⁰R¹¹, R¹⁰ and R¹¹ are selected from H, C₁-C₆ hydroxyalkyl, esterified C₁-C₆ hydroxyalkyl, C₁-C₆ alkoxyalkyl, or structural formula XIV and R¹⁰ and R¹¹ are not simultaneously H; 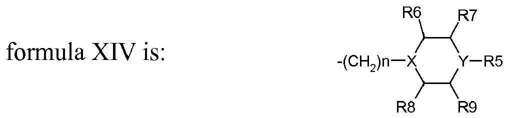 wherein R5 is selected from H, C₁-C₆ alkyl, C₁-C₆ haloalkyl, C₁-C₆ hydroxyalkyl, and C₂-C₆ alkenyl; R6-R9 are selected from H, C₁-C₆ alkoxyl, =O, C₁-C₄ alkyl, C₁-C₄ haloalkyl, C₁-C₄ hydroxyalkyl, and C₂-C₄ alkenyl; X is selected from N and CH; Y is selected from N, O,and CH; and when Y is O, then R5 is absent; and n is 1-6; and pharmaceutically acceptable salts thereof.

2. A 1-(substituted benzyl)-5-trifluoromethyl-2(1H)pyridone compound according to claim 1, wherein the pharmaceutically acceptable salts are hydrochlorate, sulfate, phosphate, perchlorate, methanesulfonate, trifluoromethanesulfonate, formate, acetate, propionate, butyrate, maleate, succinate, trifluoroacetate, succinate, salicylate, DL-aspartate, D-aspartate, L-aspartate, DL-glutamate, D-glutamate, L-glutamate, glycerate, succinate, stearate, DL-tartrate, D-tartrate, L-tartrate, (+/-)-mandelate, (R)-(-)-mandelate, (S)-(+)-mandelate, citrate, mucate, maleate, malonate, benzoate, DL-malate, D-malate, L-malate, hemimalate, 1-adamantane acetate, 1-adamantane carboxylate, flavianate, sulfoacetate, (+/-)-lactate, L-(+)-lactate, D-(-)-lactate, pamoate, D-α-galacturonic acid salt, glycerate, DL-cystine salt, D-cystine salt, L-cystine salt, DL-homocystine salt, D-homocystine salt, L-homocystine salt, DL-cysteine salt, D-cysteine salt, L-cysteine salt, (4S)-hydroxy-L-proline, cyclopropane-1,1-dicarboxylate, 2,2-methyl malonate, tyrosine salt, proline salt, fumarate, 1-hydroxy-2-naphthoate, phosphonoacetate, carbonate, bicarbonate, 3-phosphonopropionate, DL-pyroglutamate, D-pyroglutamate, L-pyroglutamate, toluenesulfonate, benzenesulfonate, esilate, (+/-)-camsilate, naphthalenesulfenesulfonate, 1R-(-)-camsilate, 1S-(+)-camsilate, 1,5-napadisilate, 1,2-ethanedisulphonate, 1,3-propanedisulphonate, 3-(N-morpholino) propane sulphonate, biphenyl sulphonate, isethionate, 1-hydroxy-2-naphthalenesulfenesulfonate, dihydric phosphate, potassium hydrogen phosphate, dipotassium phosphate, potassium phosphate, sodium hydrogen phosphate, disodium phosphate, sodium phosphate, sodium dihydrogen phosphate, calcium phosphate, tertiary calcium phosphate, hexafluoro phosphate, ethenyl phosphate, 2-carboxylethyl phosphate or phenyl phosphate.

3. 1-(substituted benzyl)-5-trifluoromethyl-2(1H)pyridone compounds according to claim 1, wherein one of R1-R4 and R12 is NR¹⁰R¹¹ or OR¹³.

4. 1-(substituted benzyl)-5-trifluoromethyl-2(1H)pyridone compounds according to claim I, wherein others are H if one of R1∼R4 and R12 is NR¹⁰R¹¹ or OR¹³.

5. 1-(substituted benzyl)-5-trifluoromethyl-2(1H)pyridone compounds according to claim 1, wherein the compounds are:
1-(4-nitrobenzyl)-5-(trifluoromethyl)pyridin-2(1H)-one;
1-(4-((3-morpholinylpropyl)amino)benzyl)-5-(trifluoromethyl)pyridin-2(1H)-one;
1-(4-(((3-piperidin-1-yl)propyl)amino)benzyl)-5-(trifluoromethyl)pyridin-2(1H)-one;
1-(4-((3-(4-methyl-piperazin-1-yl)propyl)amino)benzyl)-5-(trifluoromethyl)pyridin-2 (1H)-one;
1-(4-((2-hydroxyethyl)amino)benzyl)-5-(trifluoromethyl)pyridin-2(1H)-one;
1-(4-((2-(piperidyl-1-yl)ethyl)amino)benzyl)-5-(trifluoromethyl)pyridin-2(1H)-one;
1-(4-((2-morpholinylethyl)amino)benzyl)-5-(trifluoromethyl)pyridin-2(1H)-one;
1-(4-((2-(4-methyl-piperazin-1-yl)ethyl)amino)benzyl)-5-(trifluoromethyl)pyridin-2(1 H)-one;
1-(4-((2-(piperazin-1-yl)ethyl)amino)benzyl)-5-(trifluoromethyl)pyridin-2(1H)-one;
1-(4-((2-(4-(2-hydroxyethyl)piperazin-1-yl)ethyl)amino)benzyl)-5-(trifluoromethyl)py ridin-2(1H)-one;
1-(4-((2-(4-methyl-piperazin-1-yl)ethyl)amino)benzyl)-5-(trifluoromethyl)pyridin-2(1 H)-one dihydrochloride;
1-(2,6-dichlorobenzyl)-5-(trifluoromethyl)pyridin-2(1H)-one;
1-(4-fluorobenzyl)-5-(trifluoromethyl)pyridin-2(1H)-one;
1-(2-nitrobenzyl)-5-(trifluoromethyl)pyridin-2(1H)-one;
1-(3-chlorobenzyl)-5-(trifluoromethyl)pyridin-2(1H)-one;
1-(4-methoxybenzyl)-5-(trifluoromethyl)pyridin-2(1H)-one;
1-(2-fluorobenzyl)-5-(trifluoromethyl)pyridin-2(1H)-one;
1-(2-(2-hydroxyethylamino)benzyl)-5-(trifluoromethyl)pyridin-2(1H)-one;
1-(2-(2-(piperazin-1-yl)ethylamino)benzyl)-5-(trifluoromethyl)pyridin-2(1H)-one;
1-(2-(2-(4-methylpiperazin-1-yl)ethylamino)benzyl)-5-(trifluoromethyl)pyridin-2(1H) -one;
1-(2-(2-morpholinoethylamino)benzyl)-5-(trifluoromethyl)pyridin-2(1H)-one;
1-(4-(2-(2-hydroxyethoxy)ethylamino)benzyl)-5-(trifluoromethyl)pyridin-2(1H)-one;

6. 1-(substituted benzyl)-5-trifluoromethyl-2(1H)pyridone compounds, wherein the compounds are 1-(4-acetamide-benzyl)-5-(trifluoromethyl)pyridin-2(1H)-one; or 1-(2-acetamide-benzyl)-5-(trifluoromethyl)pyridin-2(1H)-one, 1-(4-amino-benzyl)-5-(trifluoromethyl)pyridin-2(1H)-one or 1-(2-aminobenzyl)-5-(trifluoromethyl)pyridine -2(1H)-one.

7. A compound of any one of claims 1 to 6 for use as an anti-fibrosis medicament.

## Patentansprüche

1. 1-(Substituiertes Benzyl)-5-trifluormethyl-2(1H)pyridon-Verbindungen mit einer Strukturformel (XIII), 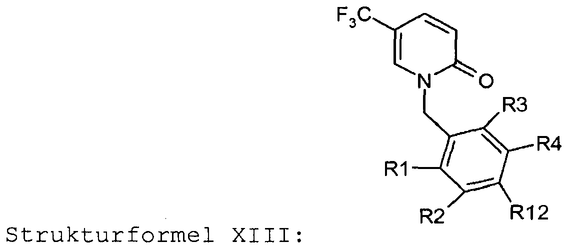 worin R1-R4, R12 ausgewählt sind aus H, NO₂ Hydroxyl, Amino, Halogenatom, C₁-C₆-Alkoxyl, NR¹⁰R¹¹, OR¹³, C(O)R¹⁴, O-C(O)R¹⁴, C₁-C₆-Alkyl, C₁-C₆-Haloalkyl, C₂-C₆-Alkenyl; worin R1∼R4, R12 nicht gleichzeitig H sind, R¹⁴ ausgewählt ist aus C₁-C₆-Alkyl; R¹³ ausgewählt ist aus Hydroxyalkyl oder Alkoxyalkyl; worin R¹⁰ und R¹¹ in NR¹⁰R¹¹ ausgewählt sind aus H, C₁-C₆-Hydroxyalkyl, verestertem C₁-C₆-Hydroxyalkyl, C₁-C₆-Alkoxyalkyl oder Strukturformel XIV, und R¹⁰ und R¹¹ nicht gleichzeitig H sind;
wobei Strukturformel XIV: 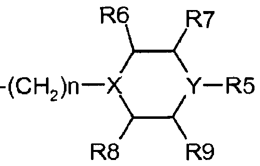 ist;
worin R5 ausgewählt ist aus H, C₁-C₆-Alkyl, C₁-C₆-Haloalkyl, C₁-C₆-Hydroxyalkyl und C₂-C₆-Alkenyl; R6-R9 ausgewählt sind aus H, C₁-C₆-Alkoxyl, =O, C₁-C₄-Alkyl, C₁-C₄-Haloalkyl, C₁-C₄-Hydroxyalkyl, und C₂-C₄-Alkenyl; X ausgewählt ist aus N und CH₂; Y ausgewählt ist aus N, O und CH; und wenn Y O ist, R5 fehlt; und n 1-6 ist; und pharmazeutisch unbedenkliche Salze davon.

2. 1-(Substitutiertes Benzyl)-5-trifluormethyl-2(1H)pyridon-Verbindung nach Anspruch 1, wobei die pharmazeutisch unbedenklichen Salze Hydrochlorat, Sulfat, Phosphat, Perchlorat, Methansulfonat, Trifluormethansulfonat, Format, Acetat, Propionat, Butyrat, Maleat, Succinat, Trifluoracetat, Succinat, Salicylat, DL-Aspartat, D-Aspartat, L-Aspartat, DL-Glutamat, D-Glutamat, L-Glutamat, Glycerat, Succinat, Stearat, DL-Tartrat, D-Tartrat, L-Tartrat, (+/-)-Mandelat, (R)-(-)-Mandelat, (S)-(+)-Mandelat, Citrat, Mucat, Maleat, Malonat, Benzoat, DL-Malat, D-Malat, L-Malat, Hemimalat, 1-Adamantanacetat, 1-Adamantancarboxylat, Flavianat, Sulfoacetat, (+/-)-Lactat, L-(+)-Lactat, D-(-)-Lactat, Pamoat, D-α-Galacturonsäuresalz, Glycerat, DL-Cystinsalz, D-Cystinsalz, L-Cystinsalz, DL-Homocystinsalz, D-Homocystinsalz, L-Homocystinsalz, DL-Cysteinsalz, D-Cysteinsalz, L-Cysteinsalz, (4S)-Hydroxy-L-prolin, Cyclopropan-1,1-dicarboxylat, 2,2-Methylmalonat, Tyrosinsalz, Prolinsalz, Fumarat, 1-Hydroxy-2-naphthoat, Phosphonacetat, Carbonat, Bicarbonat, 3-Phosphonopropionat, DL-Pyroglutamat, D-Pyroglutamat, L-Pyroglutamat, Toluolsulfonat, Benzolsulfonat, Esilat, (+/-)-Camsilat, Naphthalinsulfensulfonat, 1R-(-)-Camsilat, 1S-(+)-Camsilat, 1,5-Napadisilat, 1,2-Ethandisulphonat, 1,3-Propandisulphonat, 3-(N-Morpholino)propansulphonat, Biphenylsulphonat, Isethionat, 1-Hydroxy-2-naphthalinsulfensulfonat, Dihydrogenphosphat, Kaliumhydrogenphosphat, Dikaliumphosphat, Kaliumphosphat, Natriumhydrogenphosphat, Dinatriumphosphat, Natriumphosphat, Natriumdihydrogenphosphat, Kalziumphosphat, tertiäres Kalziumphosphat, Hexafluorphosphat, Ethenylphosphat, 2-Carboxylethylphosphat oder Phenylphosphat sind.

3. 1-(Substitutiertes Benzyl-5-trifluormethyl-2(1H)pyridon-Verbindungen nach Anspruch 1, wobei eines von R1-R4 und R12 NR¹⁰R¹¹ oder OR¹³ ist.

4. 1-(Substitutiertes Benzyl-5-trifluormethyl-2(1H)pyridon-Verbindungen nach Anspruch 1, wobei andere H sind, wenn eines von R1∼R4 und R12 NR¹⁰R¹¹ oder OR¹³ ist.

5. 1-(Substitutiertes Benzyl-5-trifluormethyl-2(1H)pyridon-Verbindungen nach Anspruch 1, wobei die Verbindungen sind:
1-(4-Nitrobenzyl)-5-(trifluormethyl)pyridin-2(1H)-on;
1-(4-((3-Morpholinylpropyl)amino)benzyl)-5-(trifluormethyl)pyridin-2(1H)-on;
1-(4-(((3-Piperidin-1-yl)propyl)amino)benzyl)-5-(trifluormethyl)pyridin-2(1H)-on;
1-(4-((3-(4-Methyl-piperazin-1-yl)propyl)amino)benzyl)-5-(trifluormethyl)pyridin-2 (1H) -on;
1-(4-((2-Hydroxyethyl)amino)benzyl)-5-(trifluormethyl)pyridin-2(1H)-on;
1-(4-((2-(Piperidyl-1-yl)ethyl)amino)benzyl)-5-(trifluormethyl)pyridin-2(1H)-on;
1-(4-((2-Morpholinylethyl)amino)benzyl)-5-(trifluormethyl)pyridin-2(1H)-on;
1-(4-((2-(4-Methyl-piperazin-1-yl)ethyl)amino)benzyl)-5-(trifluormethyl)pyridin-2(1H)-on;
1-(4-((2-(Piperazin-1-yl)ethyl)amino)benzyl)-5-(trifluormethyl)pyridin-2(1H)-on;
1-(4-((2-(4-(2-Hydroxyethyl)piperazin-1-yl)ethyl)amino)benzyl)-5-(trifluormethyl)pyridin-2(1H)-on;
1-(4-((2-(4-methyl-piperazin-1-yl)ethyl)amino)benzyl)-5-(trifluormethyl)pyridin-2(1H)-on Dihydrochlorid;
1-(2,6-Dichlorobenzyl)-5-(trifluormethyl)pyridin-2(1H)-on;
1-(4-Fluorobenzyl)-5-(trifluormethyl}pyridin-2(1H)-on;
1-(2-Nitrobenzyl)-5-(trifluormethyl)pyridin-2(1H)-on;
1-(2-Aminobenzyl)-5-(trifluormethyl)pyridin-2(1H)-on;
1-(3-Chlorbenzyl)-5-(trifluormethyl)pyridin-2(1H)-on;
1-(4-Methoxybenzyl)-5-(trifluormethyl)pyridin-2(1H)-on;
1-(2-Fluorobenzyl)-5-(trifluormethyl)pyridin-2(1H)-on;
1-(2-(2-Hydroxyethylamino)benzyl)-5-(trifluormethyl)pyridin-2(1H)-on;
1-(2-(2-(Piperazin-1-yl)ethylamino)benzyl)-5-(trifluormethyl)pyridin-2(1H)-on;
1-(2-(2-(4-Methylpiperazin-1-yl)ethylamino)benzyl)-5-(trifluormethyl)pyridin-2(1H)-on;
1-(2-(2-Morpholinoethylamino)benzyl)-5-(trifluormethyl)pyridin-2(1H)-on;
1-(4-(2-(2-Hydroxyethoxy)ethylamino)benzyl)-5-(trifluormethyl)pyridin-2(1H)-on;

6. 1-(Substitutiertes Benzyl-5-trifluormethyl-2(1H)pyridon-Verbindungen, wobei die Verbindungen 1-(4-Acetamid-benzyl)-5-(trifluormethyl)pyridin-2-(1H)-on; oder 1-(2-Acetamid-benzyl)-5-(trifluormethyl)pyridin-2-(1H)-on, 1-(4-Aminobenzyl) - 5-(trifluormethyl)pyridin-2-(1H)-on oder 1-(2-Aminobenzyl) -5-(trifluormethyl)pyridin-2-(1H)-on sind.

7. Verbindung nach einem der Ansprüche 1 bis 6 zur Verwendung als Anti-Fibrose-Medikament.

## Revendications

1. Composés de 1-(benzyle substitué)-5-trifluorométhyl-2(1H)pyridone, ayant une formule structurelle (XIII),
Formule structurelle XIII : 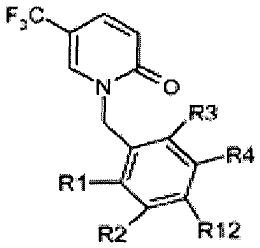 dans laquelle R1-R4, R12 sont sélectionnés à partir de H, NO₂, hydroxyle, amino, atome d'halogène, alcoxyle en C₁-C₆, NR¹⁰R¹¹, OR¹³, C(O)R¹⁴, O-C(O)R¹⁴, alkyle en C₁-C₆, halogénoalkyle en C₁-C₆, alcényle en C₂-C₆ ; dans laquelle R1-R4, R12 ne sont pas simultanément H, R¹⁴ est sélectionné à partir d'alkyle en C₁-C₆, R¹³ est sélectionné à partir d'hydroxyalkyle ou alcoxyalkyle ; dans laquelle dans NR¹⁰R¹¹, R¹⁰ et R¹¹ sont sélectionnés à partir de H, hydroxyalkyle en C₁-C₆, hydroxyalkyle en C₁-C₆ estérifié, alcoxyalkyle en C₁-C₆, ou une formule structurelle XIV et R¹⁰ et R¹¹ ne sont pas simultanément H ;
la formule XIV est : 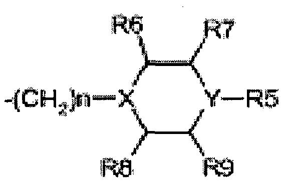 dans laquelle R5 est sélectionné à partir de H, alkyle en C₁-C₆, halogénoalkyle en C₁-C₆, hydroxyalkyle en C₁-C₆ et alcényle en C₂-C₆ ; R6-R9 sont sélectionnés à partir de H, alcoxyle en C₁-C₆, =O, alkyle en C₁-C₄, halogénoalkyle en C₁-C₄, hydroxyalkyle en C₁-C₄ et alcényle en C₂-C₄ ; X est sélectionné à partir de N et CH ; Y est sélectionné à partir de N, O et CH ; et quand Y est O, alors R5 est absent ; et n est 1-6 ; et des sels de ceux-ci acceptables d'un point de vue pharmaceutique.

2. Composé de 1-(benzyle substitué)-5-trifluorométhyl-2(1H)pyridone selon la revendication 1, dans lequel les sels acceptables d'un point de vue pharmaceutique sont de l'hydrochlorate, sulfate, phosphate, perchlorate, méthanesulfonate, trifluorométhanesulfonate, formiate, acétate, propionate, butyrate, maléate, succinate, trifluoroacétate, succinate, salicylate, DL-aspartate, D-aspartate, L-aspartate, DL-glutamate, D-glutamate, L-glutamate, glycérate, succinate, stéarate, DL-tartrate, D-tartrate, L-tartrate, (+/-)-mandélate, (R)-(-)-mandélate, (S)-(+)-mandélate, citrate, mucate, maléate, malonate, benzoate, DL-malate, D-malate, L-malate, hémimalate, acétate de 1-adamantane, carboxylate de 1-adamantane, flavianate, sulfoacétate, (+/-)-lactate, L-(+)-lactate, D-(-)-lactate, pamoate, sel d'acide D-α-galacturonique, glycérate, sel de DL-cystine, sel de D-cystine, sel de L-cystine, sel de DL-homocystine, sel de D-homocystine, sel de L-homocystine, sel de DL-cystéine, sel de D-cystéine, sel de L-cystéine, (4S)-hydroxy-L-proline, cyclopropane-1,1-dicarboxylate, malonate de 2,2-méthyle, sel de tyrosine, sel de proline, fumarate, 1-hydroxy-2-naphthoate, phosphonoacétate, carbonate, bicarbonate, 3-phosphonopropionate, DL-pyroglutamate, D-pyroglutamate, L-pyroglutamate, toluènesulfonate, benzènesulfonate, ésilate, (+/-)-camsilate, naphthalènesulfènesulfonate, 1R-(-)-camsilate, 1S-(+)-camsilate, 1,5-napadisilate, 1,2-éthanedisulphonate, 1,3-propanedisulphonate, 3-(N-morpholino) propane sulfonate, sulfonate de biphényle, iséthionate, 1-hydroxy-2-naphthalènesulfènesulfonate, phosphate dihydrique, phosphate d'hydrogène potassique, phosphate dipotassique, phosphate de potassium, phosphate d'hydrogène sodique, phosphate disodique, phosphate de sodium, dihydrogéno-phosphate de sodium, phosphate de calcium, phosphate de calcium tertiaire, hexafluoro phosphate, phosphate d'éthényle, phosphate de 2-carboxyléthyle ou phosphate de phényle.

3. Composés de 1-(benzyle substitué)-5-trifluorométhyl-2(1H)pyridone selon la revendication 1, dans lesquels un de R1-R4 et R12 est NR¹⁰R¹¹ ou OR¹³.

4. Composés de 1-(benzyle substitué)-5-trifluorométhyl-2(1H)pyridone selon la revendication 1, dans lequel d'autres sont H si un de R1-R4 et R12 est NR¹⁰R¹¹ ou OR¹³.

5. Composés de 1-(benzyle substitué)-5-trifluorométhyl-2(1H)pyridone selon la revendication 1, dans lesquels les composés sont :
1-(4-nitrobenzyl)-5-(trifluorométhyl)pyridin-2(1H)-one ;
1-(4-((3-morpholinylpropyl)amino)benzyl)-5-(trifluorométhyl)pyridin-2(1H)-one ;
1-(4-(((3-piperidin-1-yl)propyl)amino)benzyl)-5-(trifluorométhyl)pyridin-2(1H)-one ;
1-(4-((3-(4-méthyl-pipérazin-1-yl)propyl)amino)benzyl)-5-(trifluorométhyl)pyridin-2(1H)-one ;
1-(4-((2-hydroxyéthyl)amino)benzyl)-5-(trifluorométhyl)pyridin-2(1H)-one ;
1-(4-((2-(piperidyl-1-yl)éthyl)amino)benzyl)-5-(trifluorométhyl)pyridin-2(1H)-one ;
1-(4-((2-morpholinyléthyl)amino)benzyl)-5-(trifluorométhyl)pyridin-2(1H)-one ;
1-(4-((2-(4-méthyl-pipérazin-1-yl)éthyl)amino)benzyl)-5-(trifluorométhyl)pyridin-2(1H)-one ;
1-(4-((2-(pipérazin-1-yl)éthyl)amino)benzyl)-5-(trifluorométhyl)pyridin-2(1H)-one ;
1-(4-((2-(4-(2-hydroxyéthyl)pipérazin-1-yl)éthyl)amino)benzyl)-5-(trifluorométhyl)pyridin-2(1H)-one ;
dihydrochlorure de 1-(4-((2-(4-méthyl-pipérazin-1-yl)éthyl)amino)benzyl)-5-(trifluorométhyl)pyridin-2(1H)-one ;
1-(2,6-dichlorobenzyl)-5-(trifluorométhyl)pyridin-2(1H)-one ;
1-(4-fluorobenzyl)-5-(trifluorométhyl)pyridin-2(1H)-one ;
1-(2-nitrobenzyl)-5-(trifluorométhyl)pyridin-2(1H)-one ;
1-(3-chlorobenzyl)-5-(trifluorométhyl)pyridin-2(1H)-one ;
1-(4-méthoxybenzyl)-5-(trifluorométhyl)pyridin-2(1H)-one ;
1-(2-fluorobenzyl)-5-(trifluorométhyl)pyridin-2(1H)-one ;
1-(2-(2-hydroxyéthylamino)benzyl)-5-(trifluorométhyl)pyridin-2(1H)-one ;
1-(2-(2-(pipérazin-1-yl)éthylamino)benzyl)-5-(trifluorométhyl)pyridin-2(1H)-one ;
1-(2-(2-(4-méthylpipérazin-1-yl)éthylamino)benzyl)-5-(trifluorométhyl)pyridin-2(1H)-one ;
1-(2-(2-morpholinoéthylamino)benzyl)-5-(trifluorométhyl)pyridin-2(1H)-one ;
1-(4-(2-(2-hydroxyéthoxy)éthylamino)benzyl)-5-(trifluorométhyl)pyridin-2(1H)-one.

6. Composés de 1-(benzyle substitué)-5-trifluorométhyl-2(1H)pyridone, dans lesquels les composés sont 1-(4-acétamide-benzyl)-5-(trifluorométhyl)pyridin-2(1H)-one ; ou 1-(2-acétamide-benzyl)-5-(trifluorométhyl)pyridin-2(1H)-one, 1-(4-amino-benzyl)-5-(trifluorométhyl)pyridin-2(1H)-one ou 1-(2-aminobenzyl)-5-(trifluorométhyl)pyridin-2(1H)-one.

7. Composé selon l'une quelconque des revendications 1 à 6, pour une utilisation comme médicament anti-fibrose.
